# EUROPÄISCHE PATENTSCHRIFT

(11) **EP 1 140 972 B1**
(45) Veröffentlichungstag und Bekanntmachung des Hinweises auf die Patenterteilung: **05.03.2003**
(21) Anmeldenummer: 99967003.7
(22) Anmeldetag: 23.12.1999
(51) Int. Cl.: C07J 1/00, A61K 31/565, C07J 31/00, C07J 41/00, C07J 43/00, C07J 33/00, C07J 51/00

(54) **NEUE 7-ALPHA,17-ALPHA-BIS-ALKYLIERTE TESTOSTERONDERIVATE UND IHRE VERWENDUNG ZUR LANGZEITTHERAPIE VON ANDROGEN-ABHÄNGIGEN ERKRANKUNGEN**
NEW 7-ALPHA, 17-ALPHA-BIS-ALKYLATED TESTOSTERONE DERIVATIVES AND THEIR USE IN LONG-TERM THERAPY OF ANDROGEN-DEPENDENT DISEASES
NOUVEAUX DERIVES DE TESTOSTERONE 7-ALPHA, 17-ALPHA-BIS-ALKYLES ET LEUR UTILISATION POUR LA THERAPIE DE LONGUE DUREE DE MALADIES ANDROGENO-DEPENDANTES

(30) Priorität: 23.12.1998 DE 19860719
(43) Veröffentlichungstag der Anmeldung: 10.10.2001
(73) Patentinhaber: Schering Aktiengesellschaft, 13353 Berlin (DE)
(72) Erfinder: CLEVE, Arwed, D-10707 Berlin (DE); SAUER, Gerhard, D-13465 Berlin (DE); HUWE, Christoph, D-13505 Berlin (DE); PARCZYK, Karsten, D-12207 Berlin (DE); HOFFMANN, Jens, D-16567 Mühlenbeck (DE); SCHNEIDER, Martin, D-13469 Berlin (DE)
(86) Internationale Anmeldenummer: EP9910355
(87) Internationale Veröffentlichungsnummer: WO00039148

(56) Entgegenhaltungen:
- WO-A-91/00732
- WO-A-93/13122
- US-A- 3 341 557
- SOLO ET AL: "7.alpha.-Alkyltestosterone derivatives: synthesis and activity as androgens and as aromatase inhibitors" STEROIDS,US,ELSEVIER SCIENCE PUBLISHERS, NEW YORK, NY, Bd. 40, Nr. 6, Dezember 1982 (1982-12), Seiten 603-614-614, XP002111323 ISSN: 0039-128X
- CHEMICAL ABSTRACTS, vol. 101, no. 11, 10. September 1984 (1984-09-10) Columbus, Ohio, US; abstract no. 86527, DE LARMINAT, MARIE ANNE ET AL: "Synthesis and evaluation of immobilized androgens for affinity chromatography in the purification of nuclear androgen receptor" XP002134935 & PROSTATE (N. Y.) (1984), 5(2), 123-40 ,
- J. R. BROOKS ET AL: "Topical anti-androgenicity of a new 4-azasteroid in the hamster" STEROIDS: STRUCTURE, FUNCTION, AND REGULATION., Bd. 56, Nr. 8, August 1991 (1991-08), Seiten 428-433, XP002134933 ELSEVIER SCIENCE PUBLISHERS, NEW YORK, NY., US ISSN: 0039-128X
- C. LUDERSCHMIDT ET AL: "Relative Binding Affinity at Mertribolone Androgenic Binding Sites of Various Antiandrogenic Agents" ARZNEIMITTEL FORSCHUNG. DRUG RESEARCH., Bd. 37, Nr. 10, Oktober 1987 (1987-10), Seiten 1262-1265, XP002134934 EDITIO CANTOR. AULENDORF., DE ISSN: 0004-4172

## Beschreibung

Die vorliegende Erfindung betrifft neue 7α, 17α, 17β-substituierte Testosteronderivate der allgemeinen Formel I und ihre Verwendung als reine Antiandrogene zur Langzeittherapie von Androgen-abhängigen Erkrankungen, insbesondere zur Langzeit-Antiandrogentherapie des Prostatakarzinoms.

Die gegenwärtigen Therapien der Androgen-abhängigen Erkrankungen basieren auf der Reduzierung oder möglichst vollständigen Eliminierung Androgen-induzierter Effekte. Dies kann durch Blockierung der Domänen des Androgenrezeptors (AR) erfolgen, an die die Androgene als Liganden binden, oder durch Reduzierung der verfügbaren Menge an Androgenen selbst (ligand depletion). "Ligand depletion" bedeutet bei der Prostatakarzinombehandlung eine Reduzierung des Serumtestosteron-Levels testiculären Ursprungs, die entweder mittels Orchidektomie (Hodenentfernung) oder durch Hormonbehandlung mit LHRH-Analoga oder Estrogenen in hohen Dosen erreicht werden soll. Diese Therapie der Hemmung der Androgensynthese und/oder Reduzierung der Androgenkonzentration ist allerdings nur begrenzt wirksam, da man inzwischen festgestellt hat, daß selbst bei totaler Abwesenheit eines Androgens nicht-blockierte Androgenrezeptoren biologisch aktiv sein können (ligandenunabhängige AR-Aktivierung).

Als Alternative oder als Ergänzung zur "ligand depletion" wird die Antiandrogentherapie angewandt, die auf der antagonistischen Blockierung des Androgenrezeptors durch sogenannte "Antiandrogene" (nicht-steroidale oder steroidale Verbindungen) beruht. Bekannte Antiandrogene, die zur Behandlung des Prostatakarzinoms bereits klinisch genutzt werden, sind CPA (Schering AG), Flutamid (Schering Plough), Casodex (Zeneca) und Anandron® (Roussel).

Obwohl 80% der Patienten zunächst auf die vorerwähnten Therapien ansprechen, kommt es bei fast allen diesen Patienten bereits nach einer durchschnittlichen Therapiedauer von 12-18 Monaten zu einem Rückfall. Es hat sich gezeigt, daß auch die AR-Blockade durch die gegenwärtig verfügbaren Antiandrogene ungenügend ist, da diese entweder eine zu geringe Wirkstärke aufweisen und/oder sogar den Androgenrezeptor aktivieren können, also wie Androgene wirken können (Partialagonismus).

Verbindungen, die als Inhibitoren der Androgensynthese und/oder als Blocker des Androgenrezeptors wirken können, werden auch in WO91/00732 bzw. WO93/13122 beschrieben. Bei WO91/00732 handelt es sich um substituierte Steroide, die mindestens eine lange Seitenkette in einer der Positionen 6α, 7α, 14α, 15α, 16α, 17α und 17β aufweisen. Als bevorzugte Verbindungen werden EM 101, ein in 17β-Position mit Hydroxy und in 7α-Position mit einem langkettigen Alkylamid substituiertes Testosteron, und EM 150, ein in 17β-Position mit Hydroxy und in 17α-Position mit einem langkettigen lodalkin substituiertes Testosteron, beschrieben. WO93/13122 beschreibt 3-Methylsulfonylhydrazono- bzw. 3-Oximinosteroide, u.a. Testosteronderivate mit peripher antiandrogenem Wirkungsprofil. Auch diese Verbindungen weisen die vorstehend geschilderten Nachteile auf.

Zusammenfassend bleibt festzustellen, daß es gegenwärtig keine zufriedenstellende Therapie für Androgen-abhängige Erkrankungen, wie z. B. für das Prostatakarzinom, gibt, und insbesondere keine Langzeittherapie möglich ist. Die bekannten Antiandrogen-Verbindungen besitzen nicht die nötige Wirkstärke, um eine vollständige Blockierung der Androgenrezeptor-Aktivität zu gewährleisten bzw. wirken partiell agonistisch.

Die Aufgabe der vorliegenden Erfindung war es deshalb, potente antiandrogene Verbindungen bereitzustellen, die eine Langzeittherapie Androgen-abhängiger Erkrankungen ermöglichen. Insbesondere soll mit diesen Verbindungen das Prostatakarzinom wirksam behandelt werden können.

Die Aufgabe der vorliegenden Erfindung wird durch neue 7α-, 17α-, 17β-substituierte Testosteronderivate der allgemeinen Formel I gelöst 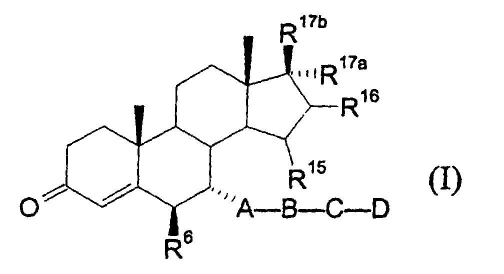 in der
- R⁶: ein Wasserstoffatom, eine Hydroxygruppe, eine C₁-C₁₀-Alkoxygruppe, eine C₁-C₁₀-Alkanoyloxygruppe oder ein Halogenatom darstellt,
- R¹⁵ und R¹⁶: je ein Wasserstoffatom sind oder gemeinsam eine Bindung bilden,
- R^{17a}: eine C₁-C₄-Alkylgruppe, eine C₂-C₄-Alkinylgruppe, oder einen Rest der Formel CₙFₘHₒ darstellt, wobei n=1,2,3 oder 4, m>1 und m+o=2n+1 ist,
- R^{17b}: eine Hydroxygruppe, eine C₁-C₁₀-Alkoxygruppe oder eine C₁-C₁₀-Alkanoyloxygruppe ist,
- A: eine unverzweigte C₆-C₁₃-Alkylengruppe ist,
- B: ein Sauerstoffatom, eine Gruppierung -S(O)ₚ-, wobei p=0,1 oder 2 ist, eine Iminocarbonylgruppe -C(O)N(Y)-, eine Iminogruppe -N(Y)-, eine Carbonyliminogruppe -N(Y)C(O)-, eine Sulfonyliminogruppe -N(Y)S(O)₂-, wobei Y ein Wasserstoffatom oder eine C₁-C₈-Alkylgruppe ist, eine Sulfonyloxygruppe -OS(O)₂-, eine Dimethylsilyloxygruppe -O-Si(CH₃)₂- oder eine Carbonylsulfanylgruppe -SC(O)- darstellt oder eine Bindung zwischen A und C darstellt oder zusammen mit C eine Bindung zwischen A und D bildet,
- C: eine Bindung zwischen B und D darstellt, oder zusammen mit B eine Bindung zwischen A und D bildet oder eine unverzweigte C₁-C₆-Alkylengruppe, eine Phenylengruppe, eine substituierte Phenylengruppe, eine Fünfring- oder Sechsring-Heteroarylengruppe, eine substituierte Fünfring- oder Sechsring-Heteroarylengruppe oder eine mit einem Phenylring kondensierte Fünfring- oder Sechsring-Heteroarylengruppe ist
und
- D: ein Wasserstoffatom, eine C₁-C₄-Alkylgruppe, eine Vinylgruppe, eine C₁-C₄-Alkoxygruppe, eine C₁-C₄-Alkoxycarbonylgruppe, eine Bis(C₁-C₄-alkoxycarbonyl)methylgruppe, eine Acetyl(C₁-C₄-Alkoxycarbonyl)methylgruppe, eine Cyangruppe, eine Carboxygruppe, eine Azidgruppe, eine Hydroxygruppe, ein Halogenatom, oder einen Rest der Formel CₙFₘHₒ darstellt, wobei n=1,2,3 oder 4, m>1 und m+o=2n+1 ist.

In einer bevorzugten Ausfühmngsform der Erfindung bedeutet R^{17a} in der allgemeinen Formel I die Methyl- oder Ethylgruppe oder die Trifluormethyl- oder Pentafluorethylgruppe. Der Rest R^{17b} stellt bevorzugt die Hydroxygruppe, eine C₁-C₅-Alkoxygruppe oder eine C₁-C₃-Alkanoylgruppe dar. Ganz besonders bevorzugt bedeutet R^{17b} die Hydroxy-, Methoxy-, Ethoxy- oder Acetyloxygruppe. Für den Rest R⁶ ist ein Wasserstoffatom, die Hydroxygruppe oder ein Halogenatom bevorzugt. In einer ganz besonders bevorzugten Ausführungsfonn der Erfindung bedeutet der Rest ABCD 9-Hydroxynonyl, 7-(Acetylsulfanyl)heptyl oder 7-(4-Cyanbutoxy)heptyl.

Im Sinne der vorliegenden Erfindung handelt es sich bei den für die Gruppierung A genannten Alkylengruppen um die Heptan-1,7-diyl-, die Octan-1,8-diyl-, die Nonan-1,9-diyl-, die Decan-1,10-diyl-, die Undecan-1,11-diyl-, die Dodecan-1,12-diyl- und die Tridecan-1,13-diyl-Gruppe. Entsprechendes gilt für die als Gruppierung C definierten Alkylengruppen.

Die für die Substituenten Y und D erwähnten Alkylgruppen stehen sowohl für die unverzweigten Gruppen, also die Methyl-, die Ethyl-, die Propylgruppe und die entsprechenden höheren Homologen, soweit sie beansprucht sind, als auch für die verzweigten Vertreter der genannten Kohlenstoffatom anzahlen, z.B. die 1-Methylethyl-, die 1-Methylpropyl-, die 2-Methylpropyl-, die 1,1-Dimethylethyl-Gruppe und so weiter. Darüber hinaus sollen auch cyclische Substituenten unter Alkylgruppen verstanden werden, je nach genannter Kohlenstoffatomanzahl z.B. der Cyclopropyl-, der Cyclopropylmethyl-, der Cyclobutyl-, der Cyclopentyl-, der Methylcyclopentyl-, der Cyclopentylmethyl- und der Cyclohexyl-Rest.

Alkoxygruppen sind die um ein Sauerstoffatom verlängerten, von den vorstehend genannten Alkylgruppen abgeleiteten Reste, also z.B. der Methoxy-, der Ethoxy-, der Propoxy-, der 1-Methylethoxy-, der 1-Methylpropoxy-, der 2-Methylpropoxy- und der 1,1-Dimethylethoxy- Rest.

Unter Alkanoyloxygruppe werden im Sinne der vorliegenden Erfindung mit verzweigten und unverzweigten Carbonsäuren der genannten Kohlenstoffatomanzahlen veresterte Hydroxygruppen verstanden, also z.B. der Formyloxy-, der Acetyloxy-, der 1-Oxopropoxy-, der 1-Oxobutoxy-, der 2-Methyl-1-oxopropoxy-Rest.

Die für die Gruppierung C angegebenen Arylen- und Hetroarylengruppen sind an einer substituierbaren Stelle mit der Gruppierung B verknüpft und an einer anderen substituierbaren Stelle mit einem Rest D substituiert. Bevorzugte Heteroaromaten sind Pyrrol, Thiophen, Imidazol, Thiazol, Oxazol, Triazol, Thiadiazol, Indol, Benzoxazol, Benzothiazol, Pyridin, Pyrimidin. Daneben können die Arylen- oder Heteroarylengruppen mit einer Methylgruppe oder einem Halogenatom substituiert sein.

Sofern in einem der Reste ein Halogenatom als Substituent erwähnt ist, kommt hierfür ein Fluor-, Chlor-, Brom- oder Iodatom in Frage. Chlor und Fluor sind bevorzugt.

Ganz besonders bevorzugt im Sinne der Erfindung sind die folgenden Verbindungen der allgemeinen Formel I:
1. 7α-(9-Chlornonyl)-17α-methyl-3-oxoandrost-4-en-17β-yl-acetat
2. 7α-(9-Chlomonyl)-17β-hydroxy-17α-methylandrost-4-en-3-on
3. 17β-Hydroxy-7α-(9-iodnonyl)-17α-methylandrost-4-en-3-on
4. 17β-Hydroxy-7α-(9-hydroxynonyl)-17α-methylandrost-4-en-3-on
5. 7α-(10-Chlordecyl)-17β-hydroxy-17α-methylandrost-4-en-3-on
6. 17β-Hydroxy-7α-(11-hydroxyundecyl)-17α-methylandrost-4-en-3-on
7. 7α-(11-Bromundecyl)-17β-hydroxy-17α-methylandrost-4-en-3-on
8. 17β-Hydroxy-17α-methyl-7α-[7-(phenylsulfanyl)heptyl]androst-4-en-3-on
9. 17β-Hydroxy-17α-methyl-7α-[9-[(4,4,5,5,5-pentafluorpentyl)sulfanyl]nonyl]androst-4-en-3-on
10. 17β-Hydroxy-17α-methyl-7α-[9-(phenylsulfanyl)nonyl]androst-4-en-3-on
11. 7α-[9-[(5-Chlorpentyl)sulfanyl]nonyl]-17β-hydroxy-17α-methylandrost-4-en-3-on
12. 17β-Hydroxy-7α-[9-[(5-hydroxypentyl)sulfanyl]nonyl]-17α-methylandrost-4-en-3-on
13. 7α-(9-Azidononyl)-17β-hydroxy-17α-methylandrost-4-en-3-on
14. 7α-[7-(Acetylsulfanyl)heptyl]-17β-hydroxy-17α-methylandrost-4-en-3-on
15. 17β-Hydroxy-17α-methyl-7α-[7-[(4,4,5,5,5-pentafluorpentyl)sulfanyl]heptyl]androst-4-en-3-on
16. N-[7-(17β-Hydroxy-17α-methyl-3-oxoandrost-4-en-7α-yl)heptyl]pentanamid
17. 17β-Hydroxy-17α-methyl-3-oxoandrost-4-en-7α-octannitril
18. 5-[[7-(17β-Hydroxy-17α-methyl-3-oxoandrost-4-en-7α-yl)heptyl]oxy]pentannitril
19. 17β-Hydroxy-17α-methyl-7α-[9-[(4,4,5,5,5-pentafluorpentyl)sulfinyl]nonyl]androst-4-en-3-on
20. N-[9-(17β-Hydroxy-17α-methyl-3-oxoandrost-4-en-7α-yl)nonyl]methansulfonamid
21. 7α-(9-Chlomonyl)-6β-hydroxy-17α-methyl-3-oxoandrost-4-en-17β-yl-acetat

Die Herstellung der erfindungsgemäßen Verbindungen erfolgt analog den in der Sterin- und Steroidliteratur umfassend beschriebenen Synthesewegen. Folgende Bücher bilden die Grundlage der Steroidsynthese: L.F. Fieser & M. Fieser: Steroids: Reinhold Publishing Corporation, NY 1959; Rood's Chemistry of Carbon Compounds (editor: S. Coffrey): Elsevier Publishing Company, 1971; und besonders das "Dictionary of Steroids" (editors: R.A. Hill; D.N. Kirk; H.L.J. Makin and G.M. Murphy): Chapmann & Hall. Letzteres beinhaltet eine ausführliche Referenzliste der Originalpublikationen bis 1990.

Die Verbindungen der vorliegenden Erfindung können nach den folgenden allgemeinen Syntheseschemata und analog den in den Beispielen angegebenen Herstellungswegen dargestellt werden. Als Ausgangsverbindung wird vorzugsweise das 3-Oxoandrosta-4,6-dien-17β-yl-acetat eingesetzt, dessen Herstellung von Bowers et al. in J. Amer. Chem. Soc. 81, 5991 (1959) beschrieben ist.

Für den Fall der Herstellung von Verbindungen mit einem Perfluoralkylrest in 17α-Position erfolgt die Ketteneinführung in 7α-Position nach Sakurai (vgl. K. Nickisch, H. Laurent, Tetrahedron Lett. 29, 1533-1536 (1988)) mit anschließender Einführung einer Carbonylschutzgruppe in Position 3 und nachfolgender Einführung des Perfluoralkylrestes in Position 17α gemäß folgendem Schema (vgl. auch Beispiele 1 - 43):

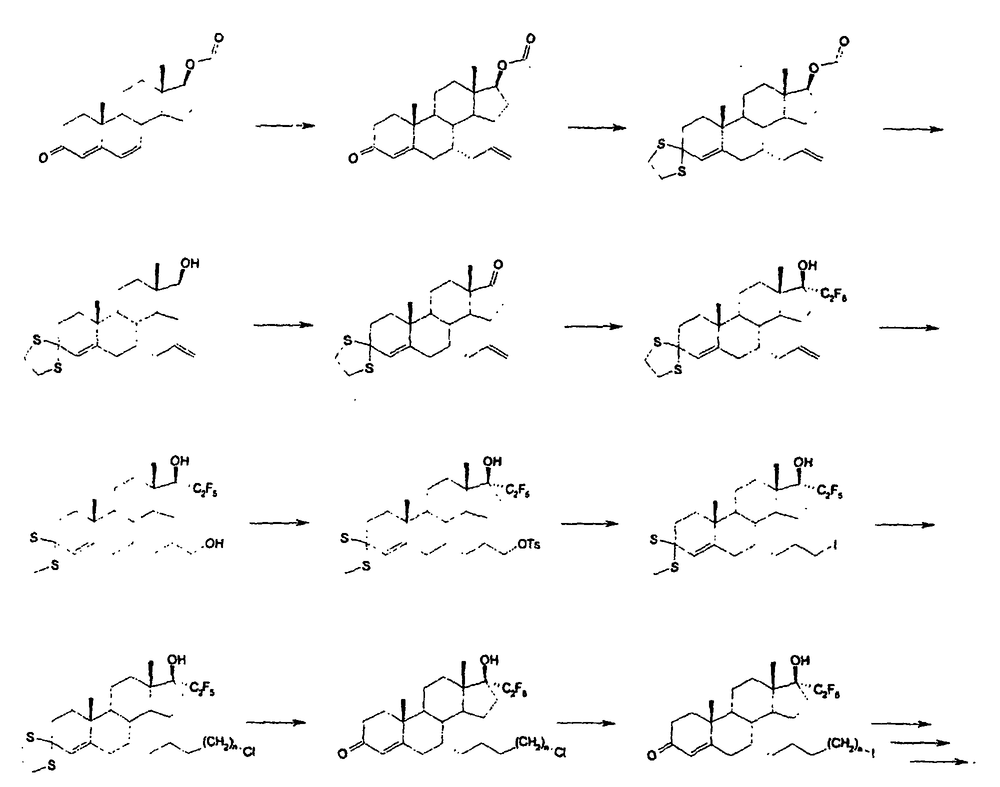

Bei der Herstellung der erfindungsgemäßen Verbindungen, die in 17α-Position eine Alkyl- oder Aikinylgruppe aufweisen, kann die Ketteneinnihrung in 7α-Position in an sich bekannter Art und Weise mit Grignard's Reagenz gemäß nachfolgendem Schema erfolgen:

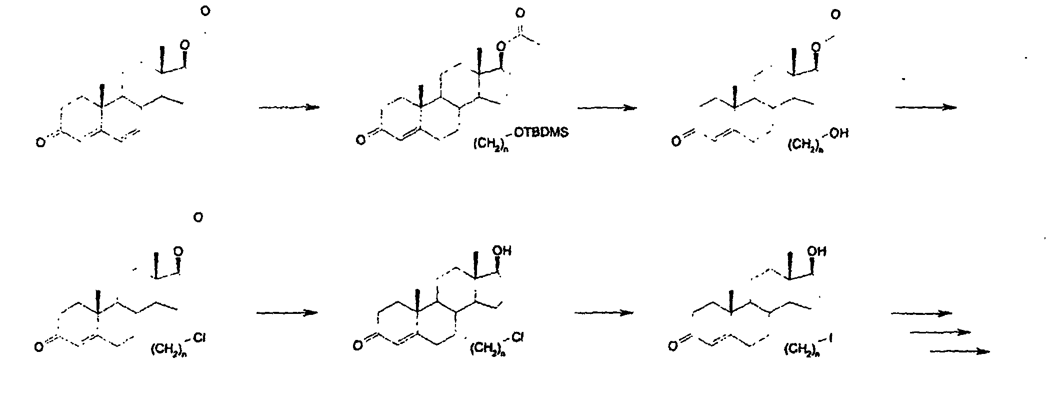

Die weitere Derivatisierung des in 7α-Position erhaltenen Alkyleniodidrestes geschieht nach üblichen organischen Synthesemethoden und kann analog den vorliegenden Beispielen vorgenommen werden.

Es wurde nun gefunden, daß die erfindungsgemäßen Verbindungen der allgemeinen Formel I als reine Antiandrogene wirken und somit die Androgenrezeptor-Aktivität vollständig blockieren. Die Verbindungen hemmen das androgenstimulierte Wachstum der humanen Prostatakarzinomzellinie LNCaP komplett. Die erfindungsgemäßen Verbindungen sind somit zur Langzeit-Antiandrogentherapie von Androgen-abhängigen Erkrankungen wie beispielsweise des Prostatakarzinoms, von Acne vulgaris, Hirsutismus, Frühpubertät, Sexualdeviationen, androgener Alopezie, gutartiger prostatischer Hyperplasie oder Seborrhöe geeignet.

Gegenstand der Erfindung ist deshalb auch die Verwendung der erfindungsgemäßen Verbindungen der allgemeinen Formel I und der als bevorzugt genannten Verbindungen zur Langzeit-Antiandrogentherapie von Androgen-abhängigen Erkrankungen, insbesondere des Prostatakarzinoms.

Die erfindungsgemäßen Verbindungen werden als pharmazeutische Zusammensetzungen verabreicht, die eine therapeutisch wirksame Menge einer oder mehrerer Verbindungen der allgemeinen Formel I beinhalten sowie ggf. galenische Hilfs- und/oder Trägerstoffe, die eine orale oder parenterale Applikation des Mittels erlauben. Die Präparate werden in Dosen von 1 - 2000 mg, bevorzugt 5 - 1000 mg, pro Applikation verabreicht. Gegenstand der Erfindung sind deshalb auch pharmazeutische Mittel, die mindestens ein Testosteronderivat der allgemeinen Formel I beinhalten.

Nachfolgend soll die Erfindung an Ausführungsbeispielen näher erläutert werden:

### Beispiel 1

### 7α-(8-Chloroctyl)-17β-hydroxy-17α-(1,1,2,2,2-pentafluorethyl)androst-4-en-3-on

### 1a) 3-Oxo-7α-(prop-2-enyl)androst-4-en-17β-yl-acetat

Zu einer Lösung von 23,11 g 3-Oxoandrosta-4,6-dien-17β-yl-acetat, dessen Herstellung in Bowers et al., *J. Amer. Chem. Soc.* **81**, 5991 (1959) beschrieben ist, in 1200 ml Dichlormethan werden bei -78°C unter Stickstoffatmosphäre langsam 38,6 ml Titantetrachlorid getropft. Nach zehn Minuten Rühren werden bei der gleichen Temperatur 67 ml Trimethyl(prop-2-enyl)silan zugetropft. Das Reaktionsgemisch wird zwei Stunden bei -78°C gerührt und bei dieser Temperatur vorsichtig mit Wasser versetzt. Die organische Phase wird nacheinander mit Wasser, gesättigter wäßriger Natriumhydrogencarbonatlösung und gesättigter wäßriger Natriumchloridlösung gewaschen, über Natriumsulfat getrocknet, filtriert und im Vakuum eingeengt. Man erhält nach Säulenchromatographie an Kieselgel mit einem Gemisch aus Hexan/Ethylacetat 14,8 g der Titelverbindung als farblosen Schaum.

¹H-NMR (CDCl₃): δ= 5,72 s (1H, H-4); 5,64 m (1H, allyl); 5,02 dbr (*J*=10 Hz, 1H, allyl); 4,99 dbr (*J*=17 Hz, 1H, allyl); 4,61 ddbr (*J*=9 Hz + 8 Hz, 1H, H-17); 2,05 s (3H, acetat); 1,20 s (3H, H-19); 0,85 s (3H, H-18).

### 1b) 3,3-[1,2-Ethandiylbis(thio))-7α-(prop-2-enyl)androst-4-en-17β-yl-acetat

4,61 g der unter 1a) hergestellten Verbindung wird in 50 ml Eisessig unter Stickstoffatmosphäre gelöst und mit 1,04 ml Ethan-1,2-dithiol sowie mit 1,18 g 4 Methylbenzolsulfonsäuremonohydrat versetzt. Das Reaktionsgemisch wird vier Stunden bei Raumtemperatur gerührt, dann auf 900 ml 2 molare wäßrige Natronlauge gegossen und mit Dichlormethan extrahiert. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 4,99 g der Titelverbindung als farblosen Schaum.

¹H-NMR (CDCl₃): δ= 5,67 ddt (*J*=17 Hz + 10 Hz + 7 Hz, 1H, allyl); 5,45 s (1H, H-4); 5,05 dbr (*J*=17 Hz, 1H, allyl); 5,01 dbr (*J*=10 Hz, 1H, allyl); 4,58 ddbr (*J*=10 Hz + 8 Hz, 1H, H-17); 3,43-3,28 m (3H, dithiolan); 3,28-3,15 m (1H, dithiolan); 2,05 s (3H, acetat); 1,04 s (3H, H-19); 0,81 s (3H, H-18).

### 1c) 3,3-[1,2-Ethandiylbis(thio)]-7α-(prop-2-enyl)androst-4-en-17β-ol

4,98 g der unter 1b) beschriebenen Verbindung werden mit 1,69 g Kaliumcarbonat in 111 ml Methanol über Nacht bei Raumtemperatur gerührt. Das Reaktionsgemisch wird weitgehend im Vakuum eingeengt. Der Rückstand wird in Wasser aufgenommen und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Man erhält 4,48 g 1c), das als Rohprodukt in die Folgestufe eingesetzt wird.

¹H-NMR (CDCl₃): δ= 5,67 ddt (*J*=17 Hz + 10 Hz + 7 Hz, 1H, allyl); 5,44 s (1H, H-4); 5,03 dbr (*J*=17 Hz, 1H, allyl); 5,01 dbr (*J*=10 Hz, 1H, allyl); 3,64 m (1H, H-17); 3,45-3,29 m (3H, dithiolan); 3,29-3,15 m (1H, dithiolan); 1,05 s (3H, H-19); 0,77 s (3H, H-18).

### 1d) 3,3-[1,2-Ethandiylbis(thio)]-7α-(prop-2-enyl)androst-4-en-17-on

4,47 g der unter 1c) hergestellten Verbindung werden in 110 ml Toluol gelöst und mit 5,11 ml Cyclohexanon sowie mit 1,01 g Aluminiumtriisopropylat fünf Stunden am Wasserabscheider zum Rückfluß erhitzt. Zur Aufarbeitung wird mit Ethylacetat verdünnt, über Celite® filtriert und mit Ethylacetat nachgewaschen. Das Filtrat wird im Vakuum eingeengt. Säulenchromatographie des Rückstands an Kieselgel mit einem Gemisch aus Hexan/Ethylacetat ergibt 4,45 g der Titelverbindung als farblosen Schaum.

¹H-NMR (CDCl₃): δ= 5,69 ddt (*J*=17 Hz + 10 Hz + 7 Hz, 1H, allyl); 5,48 s (1H, H-4); 5,06 dbr (*J*=17 Hz, 1H, allyl); 5,04 dbr (*J*=10 Hz, 1H, allyl); 3,45-3,30 m (3H, dithiolan); 3,29-3,16 m (1H, dithiolan); 2,46 dd (*J*=18 Hz + 9 Hz, 1H, H-16); 1,06 s (3H, H-19); 0,89 s (3H, H-18).

### 1e) 3,3-[1,2-Ethandiylbis(thio)]-17α-(1,1,2,2,2-pentafluorethyl)-7α-(prop-2-enyl)androst-4-en-17β-ol

22 g 1,1,1,2,2-Pentafluor-2-iodethan werden in 100 ml Toluol bei Raumtemperatur unter Stickstoff einkondensiert und bei -78°C mit einer Lösung von 4,44 g der unter 1d) hergestellten Verbindung in 50 ml Toluol versetzt. Nach zehn Minuten werden bei der gleichen Temperatur 51 ml einer 1,5 molaren Lösung von Methyllithium-Lithiumbromid-Komplex in Diethylether so langsam zugetropft, daß die Innentemperatur -65°C nicht übersteigt. Das Reaktionsgemisch wird nacheinander jeweils eine Stunde bei -78°C und bei 0°C gerührt, dann auf gesättigte wäßrige Ammoniumchloridlösung gegossen und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Nach Säulenchromatographie an Kieselgel mit einem Gemisch aus Hexan/Ethylacetat erhält man 5,67 g der Titelverbindung als farblosen Schaum.

¹H-NMR (CDCl₃): δ= 5,66 ddt (*J*=17 Hz + 10 Hz + 7 Hz, 1H, allyl); 5,45 s (1H, H-4); 5,05 dbr (*J*=17 Hz, 1H, allyl); 5,02 dbr (*J*=10 Hz, 1H, allyl); 3,43-3,29 m (3H, dithiolan); 3,29-3,16 m (1H, dithiolan); 2,39 m (1H, H-12); 1,04 s (3H, H-19); 0,97 s (3H, H-18).

### 1f) 3,3-[1,2-Ethandiylbis(thio)]-7α-(3-hydroxypropyl)-17α-(1,1,2,2,2-pentafluorethyl)androst-4-en-17β-ol

Zu einer Lösung von 5,65 g der unter 1e) hergestellten Verbindung in 110 ml Tetrahydrofuran werden bei 0°C unter Stickstoffatmosphäre 1,1 ml einer 10 molaren Lösung von Boran-Dimethylsulfid-Komplex in Tetrahydrofuran getropft. Nach 90 Minuten werden bei 0°C 22 ml 2 molare wäßrige Natronlauge und 11 ml 30%ige wäßrige Wasserstoffperoxidlösung langsam zugetropft. Das Reaktionsgemisch wird eine Stunde bei 0°C gerührt, mit Wasser verdünnt und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 2,34 g der Titelverbindung als farblosen Schaum.

¹H-NMR (CDCl₃): δ= 5,48 s (1H, H-4); 3,64 m (2H, CH₂OH); 3,43-3,28 m (3H, dithiolan); 3,28-3,16 m (1H, dithiolan); 2,39 m (1H, H-12); 1,04 s (3H, H-19); 0,96 s (3H, H-18).

### 1g) 3-[3,3-[1,2-Ethandiylbis(thio)]-17β-hydroxy-17α-(1,1,2,2,2-pentafluorethyl)androst-4-en-7α-yl]propyl-(4-methylbenzolsulfonat)

2,3 g der unter 1f) hergestellten Verbindung werden mit 3,26 g 4-Methylbenzolsulfonylchlorid und 6 ml Triethylazan in 85 ml Dichlormethan vier Stunden bei Raumtemperatur unter Stickstoffatmosphäre gerührt. Das Reaktionsgemisch wird in gesättigte wäßrige Natriumhydrogencarbonatlösung gegossen und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 1,8 g der Titelverbindung als farblosen Schaum.

¹H-NMR (CDCl₃): δ= 7,81 d (*J*=9 Hz, 2H, aryl); 7,37 d (J=9 Hz, 2H, aryl); 5,40 s (1H, H-4); 4,06 m (2H, CH₂OTs); 3,43-3,29 m (3H, dithiolan); 3,29-3,16 m (1H, dithiolan); 2,46 s (3H, tolyl); 2,36 m (1H, H-12); 1,02 s (3H, H-19); 0,94 s (3H, H-18).

### 1h) 3,3-[1,2-Ethandiylbis(thio)]-7α-(3-iodpropyl)-17α-(1,1,2,2,2-pentafluorethyl)androst-4-en-17β-ol

1,75 g der unter 1g) hergestellten Verbindung werden mit 490 mg Natriumiodid in 25 ml Aceton über Nacht zum Rückfluß erhitzt. Das Reaktionsgemisch wird filtriert und das Filtrat im Vakuum eingeengt. Säulenchromatographie an Kieselgel mit einem Gemisch aus Hexan/Ethylacetat liefert 1,36 g der Titelverbindung als farblosen Schaum.

¹H-NMR (CDCl₃): δ= 5,48 s (1H, H-4); 3,44-3,29 m (3H, dithiolan); 3,29-3,15 m (1H, dithiolan); 3,18 t (*J*=7 Hz, 2H, CH₂I); 2,40 m (1H, H-12); 1,04 s (3H, H-19); 0,96 s (3H, H-18).

### 1i) 7α-(8-Chloroctyl)-3,3-[1,2-ethandiylbis(thio)]-17α-(1,1,2,2,2-pentafluorethyl)androst-4-en-17β-ol

Aus 214 mg Magnesiumspänen in 2,2 ml Tetrahydroftiran wird durch Zutropfen einer Lösung von 1,16 ml 1-Brom-5-chlorpentan in 6,6 ml Tetrahydrofuran bei einer Innentemperatur unterhalb von 35°C und dreißigminütigem Nachrühren eine Lösung der Grignard-Verbindung 5-Chlorpentylmagnesiumbromid hergestellt. In einem anderen Kolben wird aus 7,5 mg Lithiumchlorid und 11,8 mg wasserfreiem Kupfer(II)chlorid in 0,88 ml Tetrahydrofuran durch fünfzehnminütiges Rühren bei Raumtemperatur eine braune Lösung von Dilithiumtetrachlorocuprat hergestellt. Hierzu werden 575 mg der unter 1h) hergestellten Verbindung, gelöst in 2 ml Tetrahydrofuran, getropft. Bei -10°C wird innerhalb von einer Stunde die Grignard-Lösung zu der Steroid-Lösung getropft. Während der einstündigen Nachrührzeit kommt das Reaktionsgemisch auf 0°C, Es wird dann in gesättigte wäßrige Natriumhydrogencarbonatlösung gegossen und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Nach Säülenchromatographie an Kieselgel mit einem Gemisch aus Hexan/Ethylacetat erhält man 342 mg der Titelverbindung als farbloses Öl.

¹H-NMR (CDCl₃): δ= 5,46 s (1H, H-4); 3,54 t (*J*=7 Hz, 2H, CH₂Cl); 3,43-3,29 m (3H, dithiolan); 3,29-3,14 m (1H, dithiolan); 2,39 m (1H, H-12); 1,05 s (3H, H-19); 0,97 s (3H, H-18).

### 1j) 7a-(8-Chloroctyl)-17β-hydroxy-17α-(1,1,2,2,2-pentafluorethyl)androst-4-en-3-on

330 mg der unter 1i) hergestellten Verbindung werden in 16 ml Eisessig gelöst, mit 2,43 g Glyoxylsäure versetzt und 15 Minuten bei Raumtemperatur gerührt. Dann werden 2 ml 4 molarer wäßriger Salzsäure hinzugefügt. Nach einstündigem Rühren bei Raumtemperatur wird das Reaktionsgemisch in 500 ml 2 molare wäßrige Natronlauge getropft und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat liefert 172 mg der Titelverbindung als farbloses Öl.

¹H-NMR (CDCl₃): δ= 5,73 s (1H, H-4); 3,54 t (*J*=7 Hz, 2H, CH₂Cl); 1,21 s (3H, H-19); 1,00 s (3H, H-18).

### Beispiel 2

### 17β-Hydroxy-7α-(8-iodoctyl)-17α-(1,1,2,2,2-pentafluorethyl)androst-4-en-3-on

161 mg der unter 1j) hergestellten Verbindung werden mit 87 mg Natriumiodid in 3 ml 2-Butanon über Nacht auf 80°C erhitzt. Das Reaktionsgemisch wird mit Wasser verdünnt und mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 182 mg der Titelverbindung als farbloses Öl.

¹H-NMR (CDCl₃): δ= 5,72 s (1H, H-4); 3,19 t (*J*=7 Hz, 2H, CH₂I); 1,21 s (3H, H-19); 1,00 s (3H, H-18).

### Beispiel 3

### 17β-Hydroxy-3-oxo-17α-(1,1,2,2,2-pentafluorethyl)androst-4-en-7α-nonannitril

30 mg der unter 2) hergestellten Verbindung werden mit 9 mg Kaliumcyanid in 1 ml *N,N*-Dimethylformamid 16 Stunden bei Raumtemperatur gerührt. Das Reaktionsgemisch wird mit Wasser verdünnt und mit Ethylacetat extrahiert. Die organische Phase wird mit gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 20 mg der Titelverbindung als farbloses Öl.

¹H-NMR (CDCl₃): δ= 5,72 s (1H, H-4); 2,34 t (*J*=7 Hz, 2H, CH₂CN); 1,21 s (3H, H-19); 1,00 s (3H, H-18).

### Beispiel 4

### 17β-Hydroxy-17α-(1,1,2,2,2-pentafluorethyl)-7α-[8-(phenylsulfanyl)octyl]androst-4-en-3-on

80 mg der unter 2) hergestellten Verbindung werden mit 22 mg Natriumphenylthiolat in 1,5 ml Ethanol 16 Stunden bei 60°C gerührt. Das Reaktionsgemisch wird im Vakuum eingeengt und in Ethylacetat aufgenommen. Die organische Phase wird nacheinander mit Wasser und gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 66 mg der Titelverbindung als farbloses Öl.

¹H-NMR (CDCl₃): δ 7,36-7,24 m (4H, aryl); 7,17 ddbr (*J*=8 Hz + 8 Hz, 1H, aryl); 5,73 s (1H, H-4); 2,92 t (*J*=7 Hz, 2H, CH₂S); 1,21 s (3H, H-19); 1,00 s (3H, H-18).

### Beispiel 5

### 17β-Hydroxy-17α-(1,1,2,2,2-pentafluorethyl)-7α-[8-(phenylsulfinyl)octyl]androst-4-en-3-on

36 mg der unter 4) hergestellten Verbindung werden in 0,34 ml Tetrahydrofuran gelöst, mit einer Lösung von 55 mg Natriumperiodat in 86 µl Wasser und 0,34 ml Methanol versetzt und 18 Stunden bei Raumtemperatur gerührt. Das Reaktionsgemisch wird filtriert, mit Ethylacetat nachgewaschen und im Vakuum eingeengt. Der Rückstand wird in Ethylacetat und Wasser aufgenommen. Die organische Phase wird mit gesättigter wäßriger Natriumchloridlösung gewaschen, über Natriumsulfat getrocknet, filtriert und im Vakuum eingeengt. Nach Säulenchromatographie an Kieselgel mit einem Gemisch aus Hexan/Ethylacetat erhält man 20 mg der Titelverbindung als farbloses Öl.

¹H-NMR (CDCl₃): δ= 7,61 dbr (*J*=8 Hz, 2H, aryl); 7,57-7,45 m (3H, aryl); 5,72 s (1H, H-4); 2,79 t (*J*=7 Hz, 2H, CH₂SO); 1,21 s (3H, H-19); 1,00 s (3H, H-18).

### Beispiel 6

### 7α-[8-[(2-Chlorphenyl)sulfanyl]octyl]-17β-hydroxy-17α-(1,1,2,2,2-pentafluorethyl)androst-4-en-3-on

Zu einer Suspension von 2,1 mg 60%igem Natriumhydrid als Dispersion in Mineralöl in 1 ml N,N-Dimethylformamid werden 5,9 µl 2-Chlorbenzolthiol gegeben. Nach einer Stunde bei Raumtemperatur werden 30 mg der unter 2) hergestellten Verbindung, gelöst in 1 ml *N,N*-Dimethylförmamid hinzugegeben. Das Reaktionsgemisch wird 14 Stunden bei Raumtemperatur gerührt, mit Wasser verdünnt und mit Ethylacetat extrahiert. Die organische Phase wird mit gesättigter wäßriger Natriumchloridlösung gewaschen, über Natriumsulfat getrocknet, filtriert und im Vakuum eingeengt. Säulenchromatographie an Kieselgel mit einem Gemisch aus Hexan/Ethylacetat ergibt 17 mg der Titelverbindung als farbloses Öl.

¹H-NMR (CDCl₃): δ= 7,36 dbr (*J*=8 Hz, 1H, aryl); 7,26 dbr (*J*=8 Hz, 1H, aryl); 7,22 ddbr (*J*=8 Hz + 8 Hz, 1H, aryl); 7,09 ddbr (*J*=8 Hz + 8 Hz, 1H, aryl); 5,73 s (1H, H-4); 2,93 t (*J*=7 Hz, 2H, CH₂S); 1,21 s (3H, H-19); 1,00 s (3H, H-18).

In Analogie wurden folgende Verbindungen erhalten:

| **Beispiel** | **Produkt Reagenz** **(Vorstufe \| Verfahren)** | **Form** | **Ausbeute** **[%]** | ^{**1**}**H-NMR** **δ** |
|---|---|---|---|---|
| **7** | 17β-Hydroxy-17α-(1,1,2,2,2-pentafluorethyl)-7α-[8-[(pyridin-2-yl)sulfanyl]octyl]androst-4-en-3-on | Schaum | 41 | 8,42 dbr (*J*=5 Hz, 1H, pyridinyl); 7,47 ddd (*J*=8 Hz + 8 Hz + 2 Hz, 1H, pyridinyl); 7,17 dbr (*J*=8 Hz, 1H, pyridinyl); 6,96 ddbr (*J*=8 Hz + 5 Hz, 1H, pyridinyl); 5,72 s (1H, H-4); 3,15 t (*J*=7 Hz, 2H, CH₂S); 1,20 s (3H, H-19); 0,99 s(3H,H-18) |
| | | | | |
| | Pyridin-2-thiol (2 \| 6) | | | |
| **8** | 17β-Hydroxy-17α-(1,1,2,2,2-pentafluorethyl)-7α-[8-[(pyrimidin-2-yl)sulfanyl]octyl]androst-4-en-3-on | Öl | 31 | 8,50 d (*J*=5 Hz, 2H, pyrimidinyl); 6,95 t (*J*=5 Hz, 1H, pyrimidinyl); 5,72 s (1H, H-4); 3,14 t (*J*=7 Hz, 2H, CH₂S); 1,21 s (3H, H-19); 1,00 s (3H, H-18) |
| | | | | |
| | Pyrimidin-2-thiol (2 \| 6) | | | |
| **9** | 7α-[8-[(Benzothiazol-2-yl)sulfanyl]octyl]-17β-hydroxy-17α-(1,1,2,2,2-pentafluorethyl)androst-4-en-3-on | Öl | 53 | 7,87 dbr (*J*=8 Hz, 1H, aryl); 7,76 dbr (*J*=8 Hz, 1H, aryl); 7,41 ddbr (*J*=8 Hz + 8 Hz, 1H, aryl); 7,29 ddbr (*J*=8 Hz + 8 Hz, 1H, aryl); 5,73 s (111, H-4); 3,34 t (*J*=7 Hz, 2H, CH₂S); 1,21 s (3H, H-19); 1,00 s (3H, H-18) |
| | | | | |
| | Benzothiazol-2-thiol (2 \| 6) | | | |
| **10** | 7α-[8-[(6-Ethoxybenzothiazol-2-yl)sulfanyl]octyl]-17β-hydroxy-17α-(1,1,2,2,2-pentafluorethyl)androst-4-en-3-on | Schaum | 37 | 7,74 d (*J*=9 Hz, 1H, aryl); 7,22 d (*J*=2 Hz, 1H, aryl); 7,01 dd (*J*=9 Hz + 2 Hz, 1H, aryl); 5,73 s (1H, H-4); 4,08 q (*J*=7 Hz, 2H, OEt); 3,31 t (*J*=7 Hz, 2H, CH₂S); 1,44 t (*J*=7 Hz, 3H, OEt); 1,21 s (3H, H-19); 1,00 s (3H, H-18) |
| | | | | |
| | 6-Ethoxybenzothiazol-2-thiol (2 \| 6) | | | |
| **11** | 17β-Hydroxy-17α-(1,1,2,2,2-penta-fluorethyl)-7α-[8-[(thiazol-2-yl)sulfanyl]octyl]androst-4-en-3-on | Öl | 51 | 7,66 d (*J*=3 Hz, 1H, thiazolyl); 7,20 d (*J*=3 Hz, 1H, thiazolyl); 5,73 s (1H, H-4); 3,20 t (*J*=7 Hz, 2H, CH₂S); 1,21 s (3H, H-19); 1,00 s (3H, H-18) |
| | | | | |
| | Thiazol-2-thiol (2 \| 6) | | | |
| **12** | 17β-Hydroxy-7α-[8-[(1-methyl-1*H-* imidazol-2-yl)sulfanyl]octyl]-17α-(1,1,2,2,2-pentafluorethyl)androst-4-en-3-on | Öl | 57 | 7,05 d (*J*=1 Hz, 111, imidazolyl); 6,92 d (*J*=1 Hz, 1H, imidazolyl); 5,72 s (1H, H-4); 3,62 s (3H, Me); 3,03 t (*J*=7 Hz, 2H, CH₂S); 1,21 s (3H, H-19); 1,00 s (3H, H-18) |
| | | | | |
| | 1-Methyl-1*H*-imidazol-2-thiol (2 \| 6) | | | |
| **13** | 17β-Hydroxy-7α-[8-[(5-methyl-1,3,4-thiadiazol-2-yl)sulfanyl]octyl]-17α-(1,1,2,2,2-pentafluorethyl)androst-4-en-3-on | Öl | 60 | 5,72 s (1H, H-4); 2,72 s (3H, thiadiazolyl); 3,28 t (*J*=7 Hz, 2H, CH₂S); 1,21 s (3H, H-19); 1,00 s (3H, H-18) |
| | | | | |
| | 5-Methyl-1,3,4-thiadiazol-2-thiol (2 \| 6) | | | |
| **14** | 17β-Hydroxy-17α-(1,1,2,2,2-pentafluorethyl)-7α-[8-[(thien-2-yl)sulfanyl]octyl]androst-4-en-3-on | Öl | 20 | 7,32 dd (*J*=5 Hz + 1 Hz, 1H,thienyl); 7,10 dd (*J*=4 Hz + 1 Hz, 1H,thienyl); 6,97 dd (*J*=5 Hz + 4 Hz, 1H,thienyl); 5,72 s (1H, H-4); 2,79 t (*J*=7 Hz, 2H, CH₂S); 1,21 s (3H, H-19); 1,00 s (3H, H-18) |
| | | | | |
| | Thiophen-2-thiol (2 \| 6) | | | |
| **15** | 2,2,3,3,4,4,4-Heptafluor-*N*-[8-[17β-hydroxy-3-oxo-17α-(1,1,2,2,2-pentafluorethyl)androst-4-en-7α-yl]octyl]butanamid | Öl | 62 | 6,71 sbr (1H, NH); 5,72 s (1H, H-4); 3,38 m (2H, CH₂N); 1,21 s (3H, H-19); 1,00 s (3H, H-18) |
| | | | | |
| | 2,2,3,3,4,4,4-Heptafluorbutanamid (2 \| 6) | | | |
| **16** | 17β-Hydroxy-7α-[8-[(4-methylphenyl)sulfonyl]octyl]-17α-(1,1,2,2,2-pentafluorethyl)androst-4-en-3-on | Öl | 64 | 7,79 dbr (*J*=8 Hz, 2H, aryl); 7,36 dbr (*J*=8 Hz, 2H, aryl); 5,71 s (1H, H-4); 3,06 m (2H, CH₂SO₂); 2,45 s (3H, tolyl); 1,21 s (3H, H-19); 1,00 s (3H, H-18) |
| | | | | |
| | Natrium-4-methylbenzolsulfinat (2 \| 4) | | | |
| **17** | 17β-Hydroxy-7α-[8-[(3-methylphenyl)sulfonyl]octyl]-17α-(1,1,2,2,2-pentafluorethyl)androst-4-en-3-on | Öl | 22 | 7,62 sbr (1H, aryl); 7,61 m (1H, aryl); 7,46 m (2H, aryl); 5,72 s (1H, H-4); 3,06 m (2H, CH₂SO₂); 2,46 s (3H, tolyl); 1,21 s (3H, H-19); 1,00 s (3H, H-18) |
| | | | | |
| | Natrium-3-methylbenzolsulfinat, Herstellung siehe B. Lindberg, *Acta Chem. Scand.* **17**, 377-382 (1963) (2 \| 4) | | | |
| **18a** | 7α-(10-Bromdecyl)-3,3-[1,2-ethandiylbis(thio)]-17α-(1,1,2,2,2-pentafluorethyl)androst-4-en-17β-ol | Öl | 87 | 5,46 s (1H, H-4); 3,45-3,29 m (3H, dithiolan); 3,29-3,15 m (1H, dithiolan); 3,46 t (*J*=7 Hz, 2H, CH₂Br); 2,39 m (1H, H-12); 1,04 s (3H, H-19); 0,96 s (3H, H-18) |
| | | | | |
| | 7-Bromheptylmagnesiumbromid (1h \| 1i) | | | |
| **18b** | 7α-(10-Bromdecyl)-17β-hydroxy-17α-(1,1,2,2,2-pentafluorethyl)androst-4-en-3-on | Öl | 22 | 5,73 s (1H, H-4); 3,41 t (*J*=7 Hz, 2H, CH₂Br); 1,21 s (3H, H-19); 1,00 s (3H, H-18) |
| | | | | |
| | Glyoxylsäure/Bisessig (18a \| 1j) | | | |
| **19** | 17β-Hydroxy-17α-(1,1,2,2,2-pentafluorethyl)-7α-[10-(phenylsulfanyl)decyl]androst-4-en-3-on | Öl | 76 | 7,31 dbr (*J*=8 Hz, 2H, aryl); 7,27 ddbr (*J*=8 Hz + 8 Hz, 2H, aryl); 7,16 ddbr (*J*=8 Hz + 8 Hz, 1H, aryl); 5,73 s (1H, H-4); 2,91 t (*J*=7 Hz, 2H, CH₂S); 1,21 s (3H, H-19); 0,99 s (3H, H-18) |
| | | | | |
| | Natriumphenylthiolat (18b \| 4) | | | |
| **20** | 17β-Hydroxy-17α-(1,1,2,2,2-pentafluorethyl)-7α-[10-(phenylsulfinyl)decyl]androst-4-en-3-on | Öl | 22 | 7,61 dbr (*J*=8 Hz, 2H, aryl); 7,57-7,48 m (3H, aryl); 5,73 s (1H, H-4); 2,78 t (*J*=7 Hz, 2H, CH₂SO); 1,20 s (3H, H-19); 1,00 s (3H, H-18) |
| | | | | |
| | Natriumperiodat (19 \| 5) | | | |
| **21a** | 3,3-[1,2-Ethandiylbis(thio)]-7α-(8-iodoctyl)-17α-(1,1,2,2,2-pentafluorethyl)androst-4-en-17β-ol | Öl | 83 | 5,46 s (1H, H-4); 3,43-3,29 m (3H, dithiolan); 3,29-3,14 m (1H, dithiolan); 3,18 t (*J*=7 Hz, 2H, CH₂I); 2,39 m (1H, H-12); 1,05s s (3H, H-19); 0,97 s (3H, H-18) |
| | | | | |
| | Natriumiodid (1i \| 2) | | | |
| **21b** | 7α-(13-Clortridecyl)-3,3-[1,2-ethandiylbis(thio)]-17α-(1,1,2,2,2-pentafluorethyl)androst-4-en-17β-ol | Öl | 43 | 5,46 s (1H, H-4); 3,54 t (*J*=7 Hz, 2H, CH₂CI); 3,43-3,29 m (3H, dithiolan); 3,29-3,14 m (1H, dithiolan); 2,39 m (1H, H-12); 1,05 s (3H, H-19); 0,97 s (3H, H-18) |
| | | | | |
| | 5-Chlorpentylmagnesiumbromid (21a \| 1i) | | | |
| **21c** | 7α-(13-Clortridecyl)-17β-hydroxy-17α-(1,1,2,2,2-pentafluorethyl)androst-4-en-3-on | Öl | 72 | 5,73 s (1H, H-4); 3,54 t (*J*=7 Hz, 2H, CH₂Cl); 1,21 s (3H, H-19); 1,00 s (3H, H-18) |
| | | | | |
| | Glyoxylsäure/Eisessig (21b \| 1j) | | | |
| **22** | 17β-Hydroxy-7α-(13-iodtridecyl)-17α-(1,1,2,2,2-pentafluorethyl)androst-4-en-3-on | Öl | 86 | 5,72 s (1H, H-4); 3,19 t (*J*=7 Hz, 2H, CH₂I); 1,21 s (3H, H-19); 1,00 s (3H, H-18) |
| | | | | |
| | Natriumiodid (21c \| 2) | | | |
| **23** | 17β-Hydroxy-3-oxo-17α-(1,1,2,2,2-pentafluorethyl)androst-4-en-7α-tetradecannitril | Öl | 82 | 5,73 s (1H, H-4); 2,34 t (*J*=7 Hz, 2H, CH₂CN); 1,21 s (3H, H-19); 1,00 s (3H, H-18) |
| | | | | |
| | Kaliumcyanid (22 \| 3) | | | |
| **24** | 17β-Hydroxy-17α-(1,1,2,2,2-pentafluorethyl)-7aα-[13-(phenylsulfanyl)tridecyl]androst-4-en-3-on | Öl | 87 | 7,36-7,22 m (4H, aryl); 7,15 ddbr (*J*=8 Hz + 8 Hz, 1H, aryl); 5,73 s (1H, H-4); 2,91 t (*J*=7 Hz, 2H, CH₂S); 1,21 s (3H, H-19); 1,00 s (3H, H-18) |
| | | | | |
| | Natriumphenylthiolat (22 \| 4) | | | |
| **25** | 17β-Hydroxy-17α-(1,1,2,2,2-pentafluorethyl)-7α-[13-[(3-methylphenyl)sulfanyl]tridecyl]androst-4-en-3-on | Öl | 47 | 7,18 ddbr (*J*=8 Hz + 8 Hz, 1H, aryl); 7,14 sbr (1H, aryl); 7,12 dbr (*J*=8 Hz, 1H, aryl); 6,98 dbr (*J*=8 Hz, 1H, aryl); 5,74 s (1H, H-4); 2,91 t (*J*=7 Hz, 2H, CH₂S); 2,32 s (1H, tolyl); 1,21 s (3H, H-19); 1,00 s (3H, H-18) |
| | | | | |
| | 3-Methylbenzolthiol (22 \| 6) | | | |
| **26** | 17β-Hydroxy-17α-(1,1,2,2,2-pentafluorethyl)-7α-[13-[(pyridin-2-yl)sulfanyl]tridecyl]androst-4-en-3-on | Öl | 44 | 8,42 dbr (*J*=5 Hz, 1H, pyridinyl); 7,47 ddd (*J*=8 Hz + 8 Hz + 2 Hz, 1H, pyridinyl); 7,17 dbr (*J*=8 Hz, 1H, pyridinyl); 6,97 ddbr (*J*=8 Hz + 5 Hz, 1H, pyridinyl); 5,73 s (1H, H-4); 3,15 t (*J*=7 Hz, 2H, CH₂S); 1,21 s (3H, H-19); 0,99 s (3H, H-18) |
| | | | | |
| | Pyridin-2-thiol (22 \| 6) | | | |
| **27** | 17β-Hydroxy-17α-(1,1,2,2,2-pentafluorethyl)-7α-[13-[(pyrimidin-2-yl)sulfanyl]tridecyl]androst-4-en-3-on | Öl | 31 | 8,50 d (*J*=5 Hz, 2H, pyrimidinyl); 6,94 t (*J*=5 Hz, 1H, pyrimidinyl); 5,72 s (1H, H-4); 3,13 t (*J*=7 Hz, 2H, CH₂S); 1,21 s (3H, H-19); 1,00 s (3H, H-18) |
| | | | | |
| | Pyrimidin-2-thiol (22 \| 6) | | | |
| **28** | 17β-Hydroxy-7α-[13-[(1-methyl-1*H*-imidazol-2-yl)sulfanyl]tridecyl]-17α-(1,1,2,2,2-pentafluorethyl)androst-4-en-3-on | Öl | 27 | 7,06 sbr (1H, imidazolidinyl); 6,92 sbr (1H, imidazolidinyl); 5,73 s (1H, H-4); 3,62 s (3H, NCH₃); 3,04 t (*J*=7 Hz, 2H, CH₂S); 1,21 s (3H, H-19); 1,00 s (3H, H-18) |
| | | | | |
| | 1-Methyl-1*H*-imidazol-2-thiol (22 \| 6) | | | |
| **29** | 7α-[13-[(Benzothiazol-2-yl)sulfanyl]tridecyl]-17β-hydroxy-17α-(1,1,2,2,2-pentafluorethyl)androst-4-en-3-on | Öl | 35 | 7,87 dbr (*J*=8 Hz, 1H, aryl); 7,76 dbr (*J*=8 Hz, 1H, aryl); 7,41 ddbr (*J*=8 Hz + 8 Hz, 1H, aryl); 7,31 ddbr (*J*=8 Hz + 8 Hz, 1H, aryl); 5,75 s (1H, H-4); 3,34 t (*J*=7 Hz, 2H, CH₂S); 1,21 s (3H, H-19); 1,00 s (3H, H-18) |
| | | | | |
| | Benzothiazol-2-thiol (22 \| 6) | | | |
| **30** | 7α-[13-[(6-Ethoxybenzothiazol-2-yl)sulfanyl]tridecyl]-17β-hydroxy-17α-(1,1,2,2,2-pentafluorethyl)androst-4-en-3-on | amorph | 70 | 7,74 d (*J*=9 Hz, 1H, aryl); 7,22 d (*J*=2 Hz, 1H, aryl); 7,01 dd (*J*=9 Hz + 2 Hz, 1H, aryl); 5,73 s (1H, H-4); 4,07 q (*J*=7 Hz, 2H, OEt); 3,30 (*J*=7 Hz, 2H, CH₂S); 1,44 t (*J*=7 Hz, 3H, OEt); 1,21 s (3H, H-19); 1,00 s (3H, H-18) |
| | | | | |
| | 6-Ethoxybenzothiazol-2-thiol (22 \| 6) | | | |
| **31** | 17β-Hydroxy-17α-(1,1,2,2,2-pentafluorethyl)-7α-[13-[(thiazol-2-yl)sulfanyl]tridecyl]androst-4-en-3-on | Öl | 85 | 7,66 d (7=3 Hz, 1H, thiazolyl); 7,20 d (*J*=3 Hz, 1H, thiazolyl); 5,73 s (1H, H-4); 3,20 t (*J*=7 Hz, 2H, CH₂S); 1,21 s (3H, H-19); 1,00 s (3H, H-18) |
| | | | | |
| | Thiazol-2-thiol (22 \| 6) | | | |
| **32** | 17β-Hydroxy-7α-[13-[(4-methylphenyl)sulfonyl]tridecyl]-17α-(1,1,2,2,2-pentafluorethyl)androst-4-en-3-on | Schaum | 51 | 7,78 dbr (*J*=8 Hz, 2H, aryl); 7,37 dbr (*J*=8 Hz, 2H, aryl); 5,73 s (1H, H-4); 3,06 m (2H, CH₂SO₂); 2,46 s (3H, tolyl); 1,21 s (3H, H-19); 1,00 s (3H, H-18) |
| | | | | |
| | Natrium-4-methylbenzolsulfinat (22 \| 4) | | | |
| **33a** | 3,3-[1,2-Ethandiylbis(thio)]-7α-(hex-5-enyl)-17α-(1,1,2,2,2-pentafluorethyl)androst-4-en-17β-ol | Schaum | 81 | 5,82 ddt (*J*=17 Hz + 10 Hz + 7 Hz, 1H, vinyl); 5,46 s (1H, H-4); 5,02 dbr (*J*=17 Hz, 1H, vinyl); 4,94 dbr (*J*=10 Hz, 1H, vinyl); 3,45-3,29 m (3H, dithiolan); 3,29-3,16 m (1H, dithiolan); 2,39 m (1H, H-12); 1,05 s (3H, H-19); 0,96 s (3H, H-18) |
| | | | | |
| | Prop-2-enylmagnesiumbromid (1h \| 1i) | | | |
| **33b** | 3,3-[1,2-Ethandiylbis(thio)]-7α(3-hydroxyhexyl)-17α-(1,1,2,2,2-pentafluorethyl)androst-4-en-17β-ol | Schaum | 69 | 5,45 s (1H, H-4); 3,64 tbr (*J*=6 Hz, 2H, CH₂OH); 3,44-3,29 m (3H, dithiolan); 3,29-3,16 m (1H, dithiolan); 2,39 m (1H, H-12); 1,04 s (3H, H-19); 0,96 s (3H, H-18) |
| | | | | |
| | Boran-Dimethylsulfid-Komplex (33a \| 1f) | | | |
| **33c** | 6-[17β-Hydroxy-3-oxo-17α-(1,1,2,2,2-pentafluorethyl)androst-4-en-7α-yl]hexyl-acetat | amorph | 66 | 5,73 s (1H, H-4); 4,05 t (*J*=7 Hz, 2H, CH₂O); 2,05 s (3H, acetat); 1,21 s (3H, H-19); 1,00 s (3H, H-18) |
| | | | | |
| | Glyoxylsäure/Eisessig (33b \| 1j) | | | |
| **34** | 17β-Hydroxy-7α-(6-hydroxyhexyl)-17α-(1,1,2,2,2-pentafluorethyl)androst-4-en-3-on | Schaum | 62 | 5,74 s (1H, H-4); 3,64 t (*J*=7 Hz, 2H, CH₂O); 1,21 s (3H, H-19); 1,00 s (3H, H-18) |
| | | | | |
| | Kaliumcarbonat/Methanol (33c \| 1c) | | | |
| **35** | 6-[17β-Hydroxy-3-oxo-17α-(1,1,2,2,2-pentafluorethyl)androst-4-en-7α-yl]hexyl-(4-methylbenzensulfonat) | Schaum | 87 | 7,79 d (*J*=8 Hz, 2H, Aryl); 7,35 d (*J*=8 Hz, 2H, aryl); 5,71 s (1H, H-4); 4,01 t (*J*=7 Hz, 2H, CH₂OTs); 2,46 s (3H, tolyl); 1,21 s (3H, H-19); 1,00 s (3H, H-18) |
| | | | | |
| | 4-Methylbenzolsulfonylchlorid (34 \| 1g) | | | |
| **36** | 17β-Hydroxy-7α-(6-iodhexyl)-17α-(1,1,2,2,2-pentafluorethyl)androst-4-en-3-on | Schaum | 92 | 5,73 s (1H, H-4); 3,19 t (*J*=7 Hz, 2H, CH₂I); 1,21 s (3H, H-19); 1,00 s (3H, H-18) |
| | | | | |
| | Natriumiodid (35 \| 2) | | | |
| **37** | 17β-Hydroxy-17α-(1,1,2,2,2-pentafluorethyl)-7α-[6-(phenylsulfanyl)hexyl]androst-4-en-3-on | Öl | 14 | 7,31 dbr (*J*=8 Hz, 211, aryl); 7,27 ddbr (*J*=8 Hz + 8 Hz, 2H, aryl); 7,16 ddbr (*J*=8 Hz + 8 Hz, 1H, aryl); 5,72 s (1H, H-4); 2,91 t (*J*=7 Hz, 2H, CH₂S); 1,20 s (3H, H-19); 1,00 s (3H, H-18) |
| | | | | |
| | Natriumphenylthiolat (36 \| 4) | | | |
| **38** | 17β-Hydroxy-17α-(1,1,2,2,2-pentafluorethyl)-7α-[6-(phenylsulfonyl)hexyl]androst-4-en-3-on | Öl | 78 | 7,91 dbr (*J*=8 Hz, 2H, aryl); 7,67 ddbr (*J*=8 Hz + 8 Hz, 1H, aryl); 7,58 ddbr (*J*=8 Hz + 8 Hz, 2H, aryl); 5,69 s (1H, H-4); 3,08 m (2H, CH₂SO₂); 1,20 s (3H, H-19); 1,00 s (3H, H-18) |
| | | | | |
| | Natriumbenzolsulfinat (36 \| 4) | | | |
| **39** | 17β-Hydroxy-17α-(1,1,2,2,2-pentafluorethyl)-7α-[6-[(pyridin-2-yl)sulfanyl]hexyl]androst-4-en-3-on | Öl | 58 | 8,42 dbr (*J*=5 Hz, 1H, pyridinyl); 7,47 ddd (*J*=8 Hz + 8 Hz + 2 Hz, 1H, pyridinyl); 7,18 dbr (*J*=8 Hz, 1H, pyridinyl); 6,97 ddbr (*J*=8 Hz +5 Hz, 1H, pyridinyl); 5,72 s (1H, H-4); 3,05 t (*J*=7 Hz, 2H, CH₂S); 1,20 s (3H, H-19); 1,00 s (3H, H-18) |
| | | | | |
| | Pyridin-2-thiol (36 \| 6) | | | |
| **40** | 17β-Hydroxy-17α-(1,1,2,2,2-pentafluorethyl)-7α-[6-[(pyrimidin-2-yl)sulfanyl]hexyl]androst-4-en-3-on | Öl | 82 | 8,50 d (*J*=5 Hz, 2H, pyrimidinyl); 6,94 t (*J*=5 Hz, 1H, pyrimidinyl); 5,72 s (1H, H-4); 3,12 t (*J*=7 Hz, 2H, CH₂S); 1,20 s (3H, H-19); 1,00 s (3H, H-18) |
| | | | | |
| | Pyrimidin-2-thiol (36 \| 6) | | | |
| **41** | 7α-[6-[(4,6-Dimethylpyrimidin-2-yl)sulfanyl]hexyl]-17β-hydroxy-17α-(1,1,2,2,2-pentafluorethyl)androst-4-en-3-on | Öl | 56 | 6,67 s (1H; pyrimidinyl); 5,73 s (1H, H-4); 3,25 t (*J*=7 Hz, 2H, CH₂S); 2,40 s (6H, Me); 1,20 s (3H, H-19); 1,00 s (3H, H-18) |
| | | | | |
| | 4,6-Dimethylpyrimidin-2-thiol (36 \| 6) | | | |
| **42** | 17β-Hydroxy-7α-[6-[(1-methyl-1*H*-imidazol-2-yl)sulfanyl]hexyl]-17α-(1,1,2,2,2-pentafluorethyl)androst-4-en-3-on | Öl | 20 | 7,05 d (*J*=1 Hz, 1H, imidazolyl); 6,92 d (*J*=1 Hz, 1H, imidazolyl); 5,71 s (1H, H-4); 3,62 s (3H, Me); 3,04 t (*J*=7 Hz, 2H, CH₂S); 1,20 s (3H, H-19); 1,00 s (3H, H-18) |
| | | | | |
| | 1-Methyl-1*H*-imidazol-2-thiol (36 \| 6) | | | |
| **43** | 17β-Hydroxy-17α-(1,1,2,2,2-pentafluorethyl)-7α-[6-[(thiazol-2-yl)sulfanyl]hexyl]androst-4-en-3-on | Öl | 68 | 7,65 d (*J*=4 Hz, 1H, thiazolyl); 7,21 d (*J*=4 Hz, 1H, thiazolyl); 5,72 s (1H, H-4); 3,20 t (*J*=7 Hz, 2H, CH₂S); 1,20 s (3H, H-19); 1,00 s (3H, H-18) |
| | | | | |
| | Thiazol-2-thiol (36 \| 6) | | | |

### Beispiel 44

### 7α-[9-[[(1,1-Dimethylethyl)dimethylsilyl]oxy]nonyl]-17α-methyl-3-oxoandrost-4-en-17β-yl-acetat

In 56 ml trockenem Tetrahydrofuran suspendiert man 2,82 g Magnesiumspäne (116 mmol) und startet die Bildung der Grignard-Verbindung mit wenig [(9-Bromnonyl)oxy](1,1-dimethylethyl)dimethylsilan, etwas Dibrommethan und einigen Körnchen Jod. Nach Anspringen gibt man tropfenweise die Lösung von insgesamt 39,0 g [(9-Bromnonyl)oxy](1,1-dimethylethyl)dimethylsilan (116 mmol) in 36 ml trockenen Tetrahydrofuran in der Weise zu, daß die Innentemperatur nicht über 35°C ansteigt. Danach wird die Lösung 15 Minuten auf 80°C erwärmt und dann bei -60°C mit einer Lösung versetzt, die aus 11,0 g Kupfer(I)iodid (58 mmol) in 54 ml trockenem Tetrahydrofuran durch Zugabe von 20,1 g Lithiumbromid (132 mmol) unter Eiskühlung bereitet und mit 21 ml 1,3-Dimethyl-3,4,5,6-tetrahydro-2(1*H*)-pyrimidinon verdünnt wurde. Die Innentemperatur soll bei der Zugabe nicht über -50°C ansteigen. Nach 15 Minuten Rühren bei -20°C wird auf -70°C abgekühlt und die Lösung von 17α-Methyl-3-oxoandrosta-4,6-dien-17β-yl-acetat (40 mmol), dessen Herstellung in V. Schwarz, *Collect. Czech. Chem. Commun.* **26**, 1958-1966 (1961) beschrieben ist, und 13 ml Chlortrimethylsilan in 60 ml trockenem Tetrahydrofuran und 16 ml 1,3-Dimethyl-3,4,5,6-tetrahydro-2(1*H*)pyrimidinon so schnell zugegeben, daß die Innentemperatur nicht über -65°C ansteigt. Die Mischung wird eine Stunde gerührt, wobei die Temperatur auf -50°C kommt und schließlich mit 16 ml Eisessig versetzt und eine weitere Stunde bei Raumtemperatur belassen. Dann wird der Ansatz mit Ethylacetat verdünnt, mit halbgesättigter wäßriger Ammoniumchloridlösung, mit 2 molarer wäßriger Ammoniaklösung und zweimal mit gesättigter wäßriger Kochsalzlösung ausgeschüttelt, die organische Phase mit Natriumsulfat getrocknet und eingedampft. Der Rückstand wird an Kieselgel mit Dichlormethan/Hexan chromatographiert, die Ausbeute beträgt 13,9 g (57% d. Th.) der Titelverbindung. Danach wurden 4g g 7β-[9-[[(1,1-Dimethylethyl)dimethylsilyl]oxy]nonyl]-17α-methyl-3-oxoandrost-4-en-17β-yl-acetat (15% d. Th.) isoliert. Beide Verbindungen sind ölig und wurden durch MS charakterisiert: ber. 600, gef. 600.

In Analogie wurden folgende Verbindungen erhalten:

| **Beispiel** | **Produkt Reagenz** **(Vorstufe \| Verfahren)** | **Form** | **Ausbeute** **[%]** | **MS** | |
|---|---|---|---|---|---|
| | | | | **ber.** | **gef.** |
| **45** | 7α-[7-[[(1,1-Dimethylethyl)dimethylsilyl]oxy]heptyl]-17α-methyl-3-oxoandrost-4-en-17β-yl-acetat | Öl | 51 | 572 | 572 |
| | | | | | |
| | [(7-Bromheptyl)oxy](1,1-dimethylethyl)dimethylsilan (17α-Methyl-3-oxoandrosta-4,6-dien-17β-yl-acetat \| 44) | | | | |
| **46** | 7α-[10-[[(1,1-Dimethylethyl)dimethylsilyl]oxy]decyl]-17α-methyl-3 -oxoandrost-4-en-17β-yl-acetat | Öl | 56 | 615 | 615 |
| | | | | | |
| | [(10-Bromdecyl)oxy](1,1-dimelylethyl)dimethylsilan (17α-Methyl-3-oxoandrosta-4,6-dien-17β-yl-acetat \| 44) | | | | |
| **47** | 7α-[11-[[(1,1-Dimethylethyl)dimethylsilyl]oxy]undecyl]-17α-methyl-3-oxoandrost-4-en-17β-yl-acetat | Öl | 60 | 629 | 629 |
| | | | | | |
| | [(11-Bromundecyl)oxy](1,1-dimethylethyl)dimethylsilan (17α-Methyl-3-oxoandrosta-4,6-dien-17β-yl-acetat \| 44) | | | | |
| **48** | 7α-[7-(4-Chlorbutoxy)heptyl]-17α-methyl-3-oxoandrost-4-en-17β-yl-acetat | Öl | 51 | 548 550 | 548 550 |
| | | | | | |
| | 1-Brom-7-(4-chlorbutoxy)heptan (17α-Methyl-3-oxoandrosta-4,6-dien-17β-yl-acetat \| 44) | | | | |

### Beispiel 49

### 7α-(9-Hydroxynonyl)-17α-methyl-3-oxoandrost-4-en-17β-yl-acetat

Man löst 13,9 g der unter 44) hergestellten Verbindung (23 mmol) in 150 ml Methanol/Tetrahydrofuran (2:1), gibt 25 ml 8%ige wäßrige Schwefelsäure zu und rührt 2 Stunden bei Raumtemperatur. Dann wird mit Ethylacetat verdünnt, mit gesättigter wäßriger Kochsalzlösung ausgewaschen und die organische Phase nach Trocknen mit Natriumsulfat eingedampft. Der Rückstand wird an Kieselgel mit Dichlormethan/Hexan chromatographiert, die Ausbeute beträgt 10,8 g (96% d. Th.) der Titelverbindung.

### Beispiel 50

### 7α-(9-Chlornonyl)-17α-methyl-3-oxoandrost-4-en-17β-yl-acetat

10,8 g der unter 49) hergestellten Verbindung werden in 100 ml Tetrachlormethan und 35 ml Acetonitril gelöst und mit 10,5 g Triphenylphosphin (40 mmol) bei Raumtemperatur 1 Stunde zur Reaktion gebracht. Anschließend wird mit Dichlormethan verdünnt, mit gesättigter wäßriger Natriumhydrogencarbonat- und Kochsalzlösung ausgeschüttelt und die organische Phase mit Natriumsulfat getrocknet und eingedampft. Der ölige Rückstand wird an Kieselgel mit Hexan/^{*t*}Butylmethylether chromatographiert, Ausbeute 10,2 g. (91% d. Th.) der Titelverbindung.

In Analogie wurden folgende Verbindungen erhalten:

| **Beispiel** | **Produkt Reagenz** **(Vorstufe \| Verfahren)** | **Form** | **Ausbeute** **[%]** | **MS** | |
|---|---|---|---|---|---|
| | | | | **ber.** | **gef.** |
| **51** | 7α-(9-Chlornonyl)-17β-hydroxy-17α-methylandrost-4-en-3-on | Öl | 54 | 462 464 | 462 464 |
| | | | | | |
| | Kaliumcarbonat/Methanol (50 \| 1c) | | | | |
| **52** | 17β-Hydroxy-7α-(9-iodnonyl)-17α-methylandrost-4-en-3-on | Öl | 80 | 554 | 554 |
| | | | | | |
| | Natriumiodid (51 \| 2) | | | | |
| **53** | 17β-Hydroxy-7α-(9-hydroxynonyl)-17α-methylandrost-4-en-3-on | Schaum | 74 | 444 | 444 |
| | | | | | |
| | Kaliumcarbonat/Methanol (49 \| 1c) | | | | |
| **54** | 7α-(7-Hydroxyheptyl)-17α-methyl-3-oxoandrost-4-en-17β-yl-acetat | Öl | 98 | 458 | 458 |
| | | | | | |
| | Schwefelsäure (45 \| 49) | | | | |
| **55** | 17β-Hydroxy-7α-(7-hydroxyheptyl)-17α-methylandrost-4-en-3-on | Schaum | 53 | 416 | 416 |
| | | | | | |
| | Kaliumcarbonat/Methanol (54 \| 1c) | | | | |
| **56** | 7α-(7-Chlorheptyl)-17α-hydroxy-17α-methylandrost-4-en-3-on | Öl | 80 | 434 436 | 434 436 |
| | | | | | |
| | Tetrachlormethan/Triphenylphosphin (55 \| 50) | | | | |
| **57** | 17β-Hydroxy-7α-(7-iodheptyl)-17α-methylandrost-4-en-3-on Natriumiodid (56 \| 2) | Fp 116°C | 87 | 526 | 526 |
| | | | | | |
| | | | | | |
| **58** | 7α-(7-Bromheptyl)-17β-hydroxy-17α-methylandrost-4-en-3-on | Öl | 55 | 479 481 | 479 481 |
| | | | | | |
| | Tetrabrommethan/Triphenylphosphin (55 \| 50) | | | | |
| **59** | 7α-(10-Hydroxydecyl)-17α-methyl-3-oxoandrost-4-en-17β-yl-acetat | Öl | 95 | 500 | 500 |
| | | | | | |
| | Schwefelsäure (46 \| 49) | | | | |
| **60** | 17β-Hydroxy-7α-(10-hydroxydecyl)-17α-methylandrost-4-en-3-on | Öl | 96 | 458 | 458 |
| | | | | | |
| | Kaliumcarbonat/Methanol (59 \| 1c) | | | | |
| **61** | 7α-(10-Chlordecyl)-17β-hydroxy-17α-methylandrost-4-en-3-on | Öl | 24 | 476 478 | 476 478 |
| | | | | | |
| | Tetrachlormethan/Triphenylphosphin (60 \| 50) | | | | |
| **62** | 7α-(11-Hydroxyundecyl)-17α-methyl-3-oxoandrost-4-en-17β-yl-acetat | Öl | 95 | 514 | 514 |
| | | | | | |
| | Schwefelsäure (47 \| 49) | | | | |
| **63** | 17β-Hydroxy-7α-(11-hydroxyundecyl)-17α-methylandrost-4-en-3-on | Öl | 49 | 472 | 472 |
| | | | | | |
| | Kaliumcarbonat/Methanol (62 \| 1c) | | | | |
| **64** | 7α-(11-Bromundecyl)-17β-hydroxy-17α-methylandrost-4-en-3-on | Öl | 86 | 535 537 | 535 537 |
| | | | | | |
| | Tetrabrommethan/Triphenylphosphin (63 \| 50) | | | | |
| **65** | 7α-[7-(4-Chlorbutoxy)heptyl]-17β-hydroxy-17α-methylandrost-4-en-3-on | Öl | 78 | 506 508 | 506 508 |
| | | | | | |
| | Kaliumcarbonat/Methanol (48 \| 1c) | | | | |
| **66** | 17β-Hydroxy-7α-[7-(4-iodbutoxy)heptyl]-17α-methylandrost-4-en-3-on | Öl | 92 | 598 | 598 |
| | | | | | |
| | Natriumiodid (65 \| 2) | | | | |
| **67** | 17β-Hydroxy-17α-methyl-7α-[7-(phenylsulfanyl)heptyl]androst-4-en-3-on | Öl | 74 | 508 | 508 |
| | | | | | |
| | Natriumphenylthiolat (57 \| 4) | | | | |
| **68** | 17β-Hydroxy-17α-methyl-3-oxoandrost-4-en-7α-decannitril | Öl | 44 | 453 | 453 |
| | | | | | |
| | Kaliumcyanid (52 \| 3) | | | | |

### Beispiel 69

### 17β-Hydroxy-17α-methyl-7α-[9-[(4,4,5,5,5-pentafluorpentyl)sulfanyl]nonyl]androst-4-en-3-on

Zu einer Lösung von 69 mg Thioessigsäure-*S*-(4,4,5,5,5-pentafluorpentyl)ester, dessen Herstellung in Li et al., *Tetrahedron Lett.* **35**, 9141-9144 (1994) beschrieben ist, (0,3 mmol) in 0,7 ml Methanol gibt man 0,07 ml einer 30%igen Lösung von Natriummethanolat in Methanol (0,33 mmol) und rührt 30 Minuten bei Raumtemperatur. Dann wird eine Lösung von 128 mg der unter 52) hergestellten Verbindung (0,23 mmol) in 2,3 ml *N,N*-Dimethylfonnamid hinzugegeben. Das Reaktionsgemisch wird über Nacht bei Raumtemperatur gerührt, mit Wasser versetzt und dreimal mit Ethylacetat extrahiert. Die organische Phase wird nacheinander mit Wasser und gesättigter wäßriger Natriumchloridlösung gewaschen, über Natriumsulfat getrocknet, filtriert und im Vakuum eingeengt. Der Rückstand wird an Kieselgel mit Ethylacetat/Hexan chromatographiert, die Ausbeute beträgt 95 mg (66% d. Th.) der Titelverbindung. MS: ber. 620, gef. 620.

In Analogie wurden folgende Verbindungen erhalten:

| **Beispiel** | **Produkt Reagenz** **(Vorstufe \| Verfahren)** | **Form** | **Ausbeute** **[%]** | **MS** | |
|---|---|---|---|---|---|
| | | | | **ber.** | **gef.** |
| **70** | 7α-[9-(Acetylsulfanyl)nonyl]-17β-hydroxy-17α-methylandrost-4-en-3-on | Öl | 99 | 502 | 502 |
| | | | | | |
| | Kaliumthioacetat (52 \| 3) | | | | |
| **71** | 17β-Hydroxy-17α-methyl-7α-[9-(pentylsulfanyl)nonyl]androst-4-en-3-on | Öl | 32 | 530 | 530 |
| | | | | | |
| | 1-Iodpentan (70 \| 69) | | | | |
| **72** | l7β-Hydroxy-17α-methyl-7α-[9-(phenylsulfanyl)nonyl]androst-4-en-3-on | Öl | 62 | 536 | 536 |
| | | | | | |
| | Natriumphenylthiolat (52 \| 4) | | | | |
| **73** | 5-[[9-(17β-Hydroxy-17α-methyl-3-oxoandrost-4-en-7α-yl)nonyl]sulfanyl]pentansäure-methylester | Öl | 39 | 574 | 574 |
| | | | | | |
| | 5-Iodpentansäure-methylester (70 \| 69) | | | | |
| **74** | 7α-[9-[(5-Chlorpentyl)sulfanyl)nonyl]-17β-hydroxy-17α-methylandrost-4-en-3-on | Öl | 42 | 564 566 | 564 566 |
| | | | | | |
| | 1-Chlor-5-iodpentan (70 \| 69) | | | | |
| **75** | 5-[[9-(17β-Hydroxy-17α-methyl-3-oxoandrost-4-en-7α-yl)nonyl]sulfanyl]pentannitril | Öl | 36 | 541 | 541 |
| | | | | | |
| | 5-Brompentannitril (70 \| 69) | | | | |
| **76a** | 7α-[9-[[5-[[(1,1-Dimethylethyl)dimethylsilyl]oxy]pentyl]sulfanyl]nonyl]-17β-hydroxy-17α-methylandrost-4-en-3-on | Öl | 98 | 661 | 661 |
| | | | | | |
| | [(5-Brompentyl)oxy](1,1-dimethylethyl)dimethylsilan (70 \| 69) | | | | |
| **76b** | 17β-Hydroxy-7α-[9-[(5-hydroxypentyl)sulfanyl]nonyl]-17α-methylandrost-4-en-3-on | Öl | 32 | 546 | 546 |
| | | | | | |
| | Schwefelsäure (76a \| 49) | | | | |
| **77** | 7α-[9-[(5-Brompentyl)sulfanyl]nonyl]-17β-hydroxy-17α-methylandrost-4-en-3-on | Öl | 28 | 609 611 | 609 611 |
| | | | | | |
| | Tetrabrommethan/Triphenylphosphin (76b \| 50) | | | | |
| **78** | 7α-(9-Azidononyl)-17β-hydroxy-17α-methylandrost-4-en-3-on | Öl | 66 | 469 | 469 |
| | | | | | |
| | Natriumazid (52 \| 3) | | | | |
| **79** | 7α-[9-(Butylmethylamino)nonyl]-17β-hydroxy-17α-methylandrost-4-en-3-on | Öl | 35 | 513 | 513 |
| | | | | | |
| | Butylmethylazan/Bis(1-methylethyl)ethylazan (52 \| 3) | | | | |
| **80** | 7α-[7-(Acetylsulfanyl)heptyl]-17β-hydroxy-17α-methylandrost-4-en-3-on | Öl | 80 | 474 | 474 |
| | | | | | |
| | Kaliumthioacetat (57 \| 3) | | | | |
| **81** | 17β-Hydroxy-17α-methyl-7α-[7-[(4,4,5,5,5-pentafluorpentyl)sulfanyl]heptyl]androst-4-en-3-on | Öl | 84 | 592 | 592 |
| | | | | | |
| | Thioessigsäure-*S*-(4,4,5,5,5-pentafluorpentyl)ester (57 \| 69) | | | | |
| **82** | 7α-[7-(Butylmethylamino)heptyl]-17β-hydroxy-17α-methylandrost-4-en-3-on | Öl | 32 | 485 | 485 |
| | | | | | |
| | Butylmethylazan/Bis(1-methylethyl)ethylazan (57 \| 3) | | | | |
| **83** | N-[7-(17β-Hydroxy-17α-methyl-3-oxoandrost-4-en-7α-yl)heptyl]pentanamid | Öl | 18 | 499 | 499 |
| | | | | | |
| | Pentanamid (57 \| 6) | | | | |
| **84** | 17β-Hydroxy-17α-methyl-3-oxoandrost-4-en-7α-octannitril | Öl | 56 | 425 | 425 |
| | | | | | |
| | Kaliumcyanid (57 \| 3) | | | | |
| **85** | 7α-(7-Azidoheptyl)-17β-hydroxy-17α-methylandrost-4-en-3-on | Öl | 77 | 441 | 441 |
| | | | | | |
| | Natriumazid (57 \| 3) | | | | |
| **86** | *N*-[7-(17β-Hydroxy-17α-methyl-3-oxoandrost-4-en-7*α*-yl)heptyl]methansulfonamid | Öl | 63 | 493 | 493 |
| | | | | | |
| | Methansulfonamid (57 \| 6) | | | | |
| **87** | 5-[[7-(17β-Hydroxy-17α-methyl-3-oxoandrost-4-en-7α-yl)heptyl]oxy]pentannitril | Öl | 80 | 497 | 497 |
| | | | | | |
| | Kaliumcyanid (66 \| 3) | | | | |
| **88** | 17β-Hydroxy-7α-[7-(4-methoxybutoxy)heptyl]-17α-methylandrost-4-en-3-on | Öl | 48 | 502 | 502 |
| | | | | | |
| | Natriummethanolat/Methanol (66 \| 4) | | | | |
| **89** | 7α-[7-[(But-3-enyl)oxy]heptyl]-17β-hydroxy-17α-methylandrost-4-en-3-on | Öl | 14 | 470 | 470 |
| | | | | | |
| | Natriummethanolat/Methanol (66 \| 4) | | | | |
| **90** | 17β-Hydroxy-17α-methyl-7α-[11-[(4,4,5,5,5-pentafluorpentyl)sulfanyl]undecyl]androst-4-en-3-on | Öl | 62 | 648 | 648 |
| | | | | | |
| | Thioessigsäure-*S*-(4,4,5,5,5-pentafluorpentyl)ester (64 \| 69) | | | | |
| **91** | 17β-Hydroxy-17α-methyl-7α-[11-(phenylsulfanyl)undecyl]androst-4-en-3-on | Öl | 75 | 564 | 564 |
| | | | | | |
| | Natriumphenylthiolat (64 \| 4) | | | | |
| **92** | 17β-Hydroxy-7α-(11-methoxyundecyl)-17α-methylandrost-4-en-3-on | Öl | 57 | 486 | 486 |
| | | | | | |
| | Natriummethanolat/Methanol (64 \| 4) | | | | |

### Beispiel 93

### 17β-Hydroxy-17α-methyl-7α-[9-[(4,4,5,5,5-pentafluorpentyl)sulfinyl]nonyl]androst-4-en-3-on

In 5 ml Dichlormethan löst man 83 mg der unter 69) hergestellten Verbindung, kühlt im Eisbad ab und gibt 32 mg 70%ige 3-Chlorperbenzoesäure zu. Nach 15 Minuten Rühren versetzt man mit gesättigter wäßriger Natriumthiosulfatlösung, rührt weiter 15 Minuten und verdünnt dann mit Dichlormethan. Die organische Phase wird mit gesättigter wäßriger Natriumhydrogencarbonatlösung und Kochsalzlösung gewaschen, mit Natriumsulfat getrocknet und eingedampft. Der Rückstand wird an Kieselgel über eine Dünnschichtplatte mit Aceton/Hexan chromatographiert, die Ausbeute beträgt 52 mg (62% d. Th.) der Titelverbindung. MS: ber. 636, gef. 636.

In Analogie wurden folgende Verbindungen erhalten:

| **Beispiel** | **Produkt Reagenz** **(Vorstufe \| Verfahren)** | **Form** | **Ausbeute** **[%]** | **MS** | |
|---|---|---|---|---|---|
| | | | | **ber.** | **gef.** |
| **94** | 17β-Hydroxy-17α-methyl-7α-[7-[(4,4,5,5,5-pentafluorpentyl)sulfinyl]heptyl]androst-4-en-3-on | Öl | 65 | 608 | 608 |
| | | | | | |
| | 3-Chlorperbenzoesäure (81 \| 93) | | | | |
| **95** | 17β-Hydroxy-17α-methyl-7α-[7-[(4,4,5,5,5-pentafluorpentyl)sulfonyl]heptyl]androst-4-en-3-on | Öl | 7 | 624 | 624 |
| | | | | | |
| | 3-Chlorperbenzoesäure (81 \| 93) | | | | |
| **96** | 17β-Hydroxy-17α-methyl-7α-[11-[(4,4,5,5,5-pentafluorpentyl)sulfinyl]undecyl]androst-4-en-3-on | Öl | 66 | 664 | 664 |
| | | | | | |
| | 3 -Chlorperbenzoesäure (90 \| 93) | | | | |
| **97** | 17β-Hydroxy-17α-methyl-7α-[11-[(4,4,5,5,5-pentafluorpentyl)sulfonyl]undecyl]androst-4-en-3-on | Öl | 12 | 680 | 680 |
| | | | | | |
| | 3-Chlorperbenzoesäure (90 \| 93) | | | | |
| **98** | 17β-Hydroxy-17α-methyl-7α-[7-(phenylsulfinyl)heptyl]androst-4-en-3-on | Öl | 57 | 524 | 524 |
| | | | | | |
| | 3-Chlorperbenzoesäure (67 \| 93) | | | | |
| **99** | 17β-Hydroxy-17α-methyl-7α-[7-(phenylsulfonyl)heptyl]androst-4-en-3-on | Öl | 26 | 540 | 540 |
| | | | | | |
| | 3-Chlorperbenzoesäure (67 \| 93) | | | | |
| **100** | 17β-Hydroxy-17α-methyl-7α-(9-sulfanylnonyl)androst-4-en-3-on | Öl | 43 | 460 | 460 |
| | | | | | |
| | Kaliumcarbonat/Methanol (70 \| 1c) | | | | |

### Beispiel 101

### 17β-Hydroxy-17α-methyl-3-oxoandrost-4-en-7α-heptansäure

416 mg der unter 55) hergestellten Verbindung (1 mmol) werden in 10 ml wasserfreiem Aceton gelöst und mit 5 ml einer 1 molaren Lösung von Jones-Reagenz (Chromatlösung) unter Eiskühlung versetzt. Nach 15 Minuten versetzt man mit gesättigter wäßriger Natriumsulfitlösung, schüttelt die saure Lösung mit Ethylacetat aus, extrahiert die organische Phase mit gesättigter wäßriger Kochsalzlösung, trocknet sie mit Natriumsulfat und dampft ein. Der Rückstand wird an Kieselgel mit Aceton/Hexan chromatographiert, die Ausbeute beträgt 78 mg (18% d. Th.) der Titelverbindung. MS: ber. 430, gef. 430.

### Beispiel 102

### N-Butyl-17β-hydroxy-N,17α-dimethyl-3-oxoandrost-4-en-7α-heptanamid

78 mg der unter 101) hergestellten Verbindung löst man in 6 ml Dichlormethan, kühlt auf -10°C ab und versetzt nacheinander mit 30 µl 4-Methylmorpholin, 30 µl Chlorameisensäure-(2-methylpropyl)ester und nach 10 Minuten mit 40 µl Butylmethylazan. Nach 1 Stunde Rühren bei Raumtemperatur verdünnt man mit Dichlormethan, extrahiert nacheinander mit 1 molarer wäßriger Schwefelsäure, gesättigter wäßriger Natriumhydrogencarbonatlösung und gesättigter Kochsalzlösung, trocknet die organische Phase mit Natriumsulfat und dampft ein. Der Rückstand wird an Kieselgel mit Aceton/Hexan chromatographiert, die Ausbeute beträgt 40 mg (45% d. Th.) der Titelverbindung. MS: ber. 499, gef. 499.

In Analogie wurden folgende Verbindungen erhalten:

| **Beispiel** | **Produkt Reagenz** **(Vorstufe \| Verfahren)** | **Form Form** | **Ausbeute Ausbeute [%]** | **MS** | |
|---|---|---|---|---|---|
| | | | | **ber.** | **gef.** |
| **103** | 17β-(Acetyloxy)-17α-methyl-3-oxoandrost-4-en-7α-nonansäure | Öl | 13 | 500 | 500 |
| | | | | | |
| | Jones-Reagenz (49 \| 101) | | | | |
| **104** | 17β-(Acetyloxy)-*N*-butyl-*N*,17α-dimethyl-3-oxoandrost-4-en-7α-nonanamid | Öl | 90 | 569 | 569 |
| | | | | | |
| | 4-Methylmorpholin/Chlorameisensäure-(2-methylpropyl)ester/Butylmethylazan (103 \| 102) | | | | |
| **105** | *N*-Butyl-17β-hydroxy*-N*,17α-dimethyl-3-oxoandrost-4-en-7α-nonanamid | Öl | 22 | 527 | 527 |
| | | | | | |
| | Kaliumcarbonat/Methanol (104 \| 1c) | | | | |
| **106** | 17β-(Acetyloxy)-17α-methyl-3-oxoandrost-4-en-7α-undecansäure | Öl | 15 | 528 | 528 |
| | | | | | |
| | Jones-Reagenz (62 \| 101) | | | | |
| **107** | 17β-(Acetyloxy)-*N*-butyl-*N*,17α-dimethyl-3-oxoandrost-4-en-7α-undecanamid | Öl | 86 | 597 | 597 |
| | | | | | |
| | 4-Methylmorpholin/Chlorameisensäure-(2-methylpropyl)ester/Butylmethylazan (106 \| 102) | | | | |
| **108** | *N*-Butyl-17β-hydroxy-*N*,17α-dimethyl-3-oxoandrost-4-en-7α-undecanamid | Öl | 35 | 555 | 555 |
| | | | | | |
| | Kaliumcarbonat/Methanol (107 \| 1c) | | | | |

### Beispiel 109

### 2-[9-(17β-Hydroxy-17α-methyl-3-oxoandrost-4-en-7α-yl)nonyl]propandisäure-diethylester

### 109a) 7α-(9-Chlornonyl)-3,3-[1,2-ethandiylbis(oxy)]-17α-methylandrost-4-en-17β-ol

In 20 ml Dichlormethan löst man 1,48 g der unter 51) hergestellten Verbindung und gibt 20 ml 1,2-Ethandiol, 12 ml Trimethoxymethan und 0,6 g Pyridinium-p-toluolsulfonat zu. Die Mischung wird über Nacht bei Raumtemperatur gerührt, dann mit Triethylazan versetzt, mit Dichlormethan verdünnt und mit Wasser und gesättigter wäßriger Kochsalzlösung ausgeschüttelt. Die organische Phase wird mit Natriumsulfat getrocknet, eingedampft und an Kieselgel mit Hexan/^{*t*}Butylmethylether chromatographiert. Die Ausbeute beträgt 1,12 g (69% d. Th.) der Titelverbindung. MS: ber. 506/508, gef. 506/508.

### 109b) 3,3-[1,2-Ethandiylbis(oxy)]-7α-(9-iodnonyl)-17α-methylandrost-4-en-17β-ol

1,09 g der unter 109a) hergestellten Verbindung werden analog zu dem in Beispiel 2) beschriebenen Verfahren mit 1,5 g Natriumiodid zu 1,37 g der Titelverbindung als farblosem Öl umgesetzt. MS: ber. 598, gef. 598

### 109c) 2-[9-(17β-Hydroxy-17α-methyl-3-oxoandrost-4-en-7α-yl)nonyl]propandisäure-diethylester

Man deprotoniert 80 mg Propandisäure-diethylester in 0,5 ml wasserfreiem Tetrahydrofuran mit 12 mg 80%igem Natriumhydrid, gibt 60 mg der unter 109b) hergestellten Verbindung (0,1 mmol) in 1 ml wasserfreiem *N,N*-Dimethylformamid zu und erwärmt 5 Stunden auf 80°C. Nach Abkühlen wird wie üblich mit Ethylacetat aufgearbeitet. Der Rückstand wird in 0,5 ml Aceton gelöst und mit 0,1 ml 4 molarer wäßriger Salzsäure 15 Minuten bei Raumtemperatur gerührt. Anschließend wird wieder mit Ethylacetat aufgearbeitet und chromatographiert. Die Ausbeute beträgt 29 mg (49% d. Th.) der Titelverbindung. MS: ber. 586, gef. 586.

In Analogie wurden folgende Verbindungen erhalten:

| **Beispiel** | **Produkt Reagenz** **(Vorstufe \| Verfahren)** | **Form** | **Ausbeute** **[%]** | **MS** | |
|---|---|---|---|---|---|
| | | | | **ber.** | **gef.** |
| **110** | 2-[2-Acetyl-9-(17β-hydroxy-17α-methyl-3-oxoandrost-4-en-7α-yl)nonyl]undecansäureethylester | Öl | 51 | 556 | 556 |
| | | | | | |
| | 3-Oxobutansäure-ethylester (109b \| 109c) | | | | |
| **111** | 17β-Hydroxy-17α-methyl-7α-[9-(pentyloxy)nonyl]androst-4-en-3-on | Öl | 23 | 514 | 514 |
| | | | | | |
| | 1-Pentanol (109b \| 109c) | | | | |
| **112** | *N*-[9-(17β-Hydroxy-17α-methyl-3-oxoandrost-4-en-7α-yl)nonyl]pentanamid | Öl | 21 | 527 | 527 |
| | | | | | |
| | Pentanamid (109b \| 109c) | | | | |
| **113** | *N*-[9-(17β-Hydroxy-17α-methyl)-3-oxoandrost-4-en-7α-yl)nonyl]methansulfonamid | Öl | 57 | 521 | 521 |
| | | | | | |
| | Methansulfonnamid (109b \| 109c) | | | | |

### Beispiel 114

### 7α-(9-Chlornonyl)-6β-hydroxy-17α-methyl-3-oxoandrost-4-en-17β-yl-acetat

Man löst 3,3 g der unter 50) hergestellten Verbindung in 22 ml 2,2-Dimethoxypropan, gibt 0,4 g Pyridinium-*p*-toluolsulfonat zu und erhitzt 22 Stunden am Rückfluß. Nach dem Abkühlen versetzt man mit Triethylazan und dampft zum Trocknen ein. Der Rückstand wird an Kieselgel mit Hexan/^{*t*}Butylmethylether chromatographiert. Man erhält 2,91 g 7α-(9-Chlornonyl)-3-methoxy-17α-methylandrosta-3,5-dien-17β-yl-acetat (84% d. Th.), das sofort weiter umgesetzt wird.

Diese Substanz wird in 60 ml einer Mischung aus Ethanol/Wasser 95:5 suspendiert, mit 1,7 g 3-Chlorperbenzoesäure (6,8 mmol) versetzt und 45 Minuten bei Raumtemperatur gerührt. Anschließend gibt man 5 ml 2 molarer wäßriger Schwefelsäure zu, rührt 15 Minuten bei Raumtemperatur und verdünnt mit Ethylacetat. Die organische Phase wird mit Wasser und gesättigten wäßrigen Lösungen von Natriumdithionat, Natriumhydrogencarbonat und Kochsalz ausgeschüttelt, mit Natriumsulfat getrocknet und eingedampft. Nach Chromatographie an Kieselgel mit Hexan/Ethylacetat erhält man 1,0 g (30% d. Th.) der Titelverbindung. MS: ber. 520/522, gef. 520/522.

In Analogie wurden folgende Verbindungen erhalten:

| **Beispiel** | **Produkt Reagenz** **(Vorstufe) Verfahren)** | **Form** | **Ausbeute** **[%]** | **MS** | |
|---|---|---|---|---|---|
| | | | | **ber.** | **gef.** |
| **115** | 6β-Hydroxy-7α-(9-hydroxynonyl)-17α-methyl-3-oxoandrost-4-en-17β-yl-acetat | Öl | 8 | 502 | 502 |
| | | | | | |
| | 3-Chlorperbenzoesäure (49 \| 114) | | | | |
| **116** | 6β,17β-Dihydroxy-7α-(7-hydroxyheptyl)-17α-methylandrost-4-en-3-on | Öl | 9 | 432 | 432 |
| | | | | | |
| | 3-Chlorperbenzoesäure (55 \| 114) | | | | |
| **117** | 6β,17β-Dihydroxy-17α-methyl-3-oxoandrost-4-en-7α-octannitril | Öl | 8 | 441 | 441 |
| | | | | | |
| | 3-Chlorperbenzoesäure (84 \| 114) | | | | |
| **118** | 7α-[7-(4-Chlorbutoxy)heptyl]-6β,17β-dihydroxy-17α-methylandrost-4-en-3-on | Öl | 14 | 522 524 | 522 524 |
| | | | | | |
| | 3-Chlorperbenzoesäure (65 \| 114) | | | | |

### Beispiel 119: Antiproliferationstest mit der humanen Prostatakarzinomzellinie LNCaP

Die humane Prostatakarzinomzellinie LNCaP [American Type Culture Collection (ATCC) - Accession No.: CRL 1740; Horoszewicz et al., *Cancer Research*, **43**, p1809-18, 1983] wurde aus der Lymphknotenmetastase eines Prostatakarzinompatienten isoliert. Sie exprimiert den Androgenrezeptor und ist im Wachstum durch Androgene stimulierbar. Die Androgenvermittelte Wachstumsstimulation kann durch gleichzeitige Gabe von Antiandrogenen blockiert werden. Über Dosis-Wirkungsbeziehungen kann die antiandrogene Wirkstärke (IC50) von Testverbindungen ermittelt werden. Kommt es bei alleiniger Gabe einer Testverbindung zu einer Wachstumsstimulation, ist dies durch eine androgene Wirkung zu erklären, die die erfindungsgemäßen Verbindungen nicht aufweisen sollen.

### Durchführung:

Die Zellen werden in RPMI 1640 Medium mit Penicillin (10000 units/l), Streptomycin (100 mg/l), Glutamin (200 mMol), 10 % Fötalem Kälberserum und 0,1 nM des synthetischen Androgens R1881 (Metribolon, Roussel) kultiviert.
Tag 1:Aussäen der Zellen in einer Dichte von 5000-6000/100µl/well in 96-Well-Platten.
Hinzufügen der Testverbindung (100µl/well doppelt konzentriert) in Kulturmedium mit 0,2 nM R1881 (ergibt 0,1 nM Endkonzentration). Inkubation der Zellen für 72 oder 96 Stunden bei 37°C, 5% CO2, 90% relative Luftfeuchtigkeit. In dem Kulturmedium ist das Fötale Kälberserum durch 5 % Aktivkohle-behandeltes (steroidfreies) Serum ersetzt.
Tag 3 oder 4: Mediumwechsel: Jeweils 50 % des Mediums werden durch frisches Medium inklusive Testverbindungen ersetzt. Inkubation der Zellen für 96 oder 72 Stunden bei 37°C, 5% CO2, 90% relative Luftfeuchtigkeit.
Tag 7: Hinzufügen von 25µl MTT-Lösung pro Well {MTT = (3[4,5-Dimethylthiazol-2-yl]-2,5-diphenaltetrazoliumbromid, Thiazolylblau}. Inkubation 3h bei 37°C, 5% CO₂, 90% relative Luftfeuchtigkeit. Nach Entfernung des Überstandes Zugabe von 100µl DMSO pro Well. Messung der optischen Dichte bei 570 nm.

Es wurden die in der klinischen Praxis befindlichen Antiandrogene OH-Flutamid und Casodex getestet sowie die Verbindung EM-101 (N-butyl, N-methyl-11-(17'β-hydroxy-4'-androsten-3'-on-7'α-yl)undecanamid aus WO 91/00732.

| Ergebnisse: | | |
|---|---|---|
| | Antiandrogenität | Androgenität |
| | IC50 in Gegenwart von 0,1 nM R1881 | Bei 1µM* |
| OH-Flutamid | > 10000 nM | 144 % |
| Casodex | 440 nM | 7 % |
| EM-101 | 440 nM | 0% |
| Beispiel 53 | 40 nM | 0 % |
| Beispiel 80 | 200 nM | 0 % |
| Beispiel 87 | 82 nM | 0 % |

| | | |
|---|---|---|
| * Die Wachstumsstimulation durch 0,1 nM R1881 wurde = 100 % gesetzt. | | |

Die Ergebnisse zeigen, daß die erfindungsgemäßen Verbindungen bei einer verbesserten antiandrogenen Wirksamkeit (niedrigere IC₅₀-Werte) keine androgene Wirkung entfalten.

## Patentansprüche

1. Testosteronderivate der allgemeinen Formel I 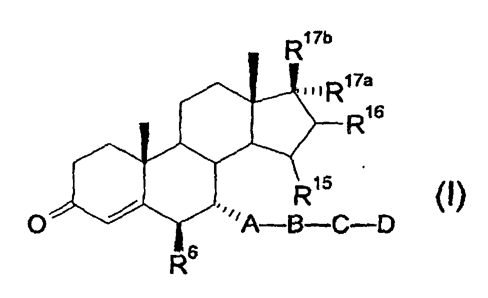 in der
R⁶ ein Wasserstoffatom, eine Hydroxygruppe, eine C₁-C₁₀-Alkoxygruppe, eine C₁-C₁₀-Alkanoyloxygruppe oder ein Halogenatom darstellt,
R¹⁵ und R¹⁶ je ein Wasserstoffatom sind oder gemeinsam eine Bindung bilden,
R^{17a} eine C₁-C₄-Alkylgruppe, eine C₂-C₄-Alkinylgruppe, oder einen Rest der Formel CₙFₘHₒ darstellt, wobei n=1,2,3 oder 4, m>1 und m+o=2n+1 ist,
R^{17b} eine Hydroxygruppe, eine C₁-C₁₀-Alkoxygruppe oder eine C₁-C₁₀-Alkanoyloxygruppe ist,
A eine unverzweigte C₆-C₁₃-Alkylengruppe ist;
B ein Sauerstoffatom, eine Gruppierung -S(O)ₚ-, wobei p=0,1 oder 2 ist, eine Iminocarbonylgruppe -C(O)N(Y)-, eine Iminogruppe -N(Y)-, eine Carbonyliminogruppe -N(Y)C(O)-, eine Sulfonyliminogruppe N(Y)S(O)₂-, wobei Y ein Wasserstoffatom oder eine C₁-C₈-Alkylgruppe ist, eine Sulfonyloxygruppe -OS(O)₂-, eine Dimethylsilyloxygruppe -O-Si(CH₃)₂- oder eine Carbonylsulfanylgruppe -SC(O)- darstellt oder eine Bindung zwischen A und C darstellt oder zusammen mit C eine Bindung zwischen A und D bildet,
C eine Bindung zwischen B und D darstellt,oder zusammen mit B eine Bindung zwischen A und D bildet oder eine unverzweigte C₁-C₆-Alkylengruppe, eine Phenylengruppe, eine substituierte Phenylengruppe, eine Fünfring- oder Sechsring-Heteroarylengruppe, eine substituierte Fünfring- oder Sechsring-Heteroarylengruppe oder eine mit einem Phenylring kondensierte Fünfring- oder Sechsring-Heteroarylengruppe ist
und
D ein Wasserstoffätom, eine C₁-C₄-Alkylgruppe, eine Vinylgruppe, eine C₁-C₄-Alkoxygruppe, eine C₁-C₄-Alkoxycarbonylgruppe, eine Bis(C₁-C₄-alkoxycarbonyl)methylgruppe, eine Acetyl(C₁-C₄-Alkoxycarbonyl)methylgruppe, eine Cyangruppe, eine Carboxygruppe, eine Azidgruppe, eine Hydroxygruppe, ein Halogenatom, oder einen Rest der Formel CₙFₘHₒ darstellt, wobei n=1,2,3 oder 4, m>1 und m+o=2n+1 ist.

2. Testosteronderivate gemäß Anspruch 1,
**dadurch gekennzeichnet, daß**
R^{17a} die Methylgruppe, die Ethylgruppe, die Trifluormethyl- oder die Pentafluorethylgruppe darstellt.

3. Testosteronderivate gemäß Anspruch 1 oder 2,
**dadurch gekennzeichnet, daß**
R^{17b} die Hydroxygruppe, eine C₁-C₅-Alkoxygruppe oder eine C₁-C₃-Alkanoyloxygruppe ist.

4. Testosteronderivate gemäß Anspruch 3,
**dadurch gekennzeichnet, daß**
R^{17b} die Hydroxy-, Methoxy-, Ethoxy- oder Acetyloxygruppe ist.

5. Testosteronderivate gemäß einem der Ansprüche 1 bis 4,
**dadurch gekennzeichnet, daß**
R⁶ ein Wasserstoffatom, die Hydroxgruppe oder ein Halogenatom darstellt.

6. Testosteronderivate gemäß einem der Ansprüche 1 bis 5,
**dadurch gekennzeichnet, daß**
R¹⁵ und R¹⁶ je ein Wasserstoffatom darstellen.

7. Testosteronderivate gemäß einem der Ansprüche 1 bis 6,
**dadurch gekennzeichnet, daß**
der Rest ABCD 9-Hydroxynonyl, 7-(Acetylsulfanyl)heptyl oder 7-(4-Cyanbutoxy)heptyl bedeutet.

8. Testosteronderivate gemäß einem der Ansprüche 1 bis 6,
**dadurch gekennzeichnet, daß**
die Fünfring- oder Sechsring-Heteroaromaten des Restes C Pyrrol, Thiophen, Imidazol, Thiazol, Oxazol, Triazol, Thiadiazol, Indol, Benzoxazol, Benzothiazol, Pyridin oder Pyrimidin sind.

9. Testosteronderivate gemäß einem der Ansprüche 1 bis 8,
**dadurch gekennzeichnet, daß**
sie die folgenden Verbindungen darstellen:
7α-(9-Chlornonyl)-17α-methyl-3-oxoandrost-4-en-17β-yl-acetat
7α-(9-Chlomonyl)-17β-hydroxy-17α-methylandrost-4-en-3-on
17β-Hydroxy-7α-(9-iodnonyl)-17α-methylandrost-4-en-3-on
17β-Hydroxy-7α-(9-hydroxynonyl)-17α-methylandrost-4-en-3-on
7α-(10-Chlordecyl)-17β-hydroxy-17α-methylandrost-4-en-3-on
17β-Hydroxy-7α-(11-hydroxyundecyl)-17α-methylandrost-4-en-3-on
7α-(11-Bromundecyl)-17β-hydroxy-17α-methylandrost-4-en-3-on
17β-Hydroxy-17α-methyl-7α-[7-(phenylsulfanyl)heptyl]androst-4-en-3-on
17β-Hydroxy-17α-methyl-7α-[9-[(4,4,5,5,5-pentafluorpentyl)sulfanyl]nonyl]androst-4-en-3-on
17β-Hydroxy-17α-methyl-7α-[9-(phenylsulfanyl)nonyl]androst-4-en-3-on
7α-[9-[(5-Chlorpentyl)sulfanyl]nonyl]-17β-hydroxy-17α-methylandrost-4-en-3-on
17β-Hydroxy-7α-[9-[(5-hydroxypentyl)sulfanyl]nonyl]-17α-methylandrost-4-en-3-on
7α-(9-Azidononyl)-17β-hydroxy-17α-methylandrost-4-en-3-on
7α-[7-(Acetylsulfanyl)heptyl]-17β-hydroxy-17α-methylandrost-4-en-3-on
17β-Hydroxy-17α-methyl-7α-[7-[(4,4,5,5,5-pentafluorpentyl)sulfanyl]heptyl]androst-4-en-3-on
N-[7-(17β-Hydroxy-17α-methyl-3-oxoandrost-4-en-7α-yl)heptyl]pentanamid
17β-Hydroxy-17α-methyl-3-oxoandrost-4-en-7α-octannitril
5-[[7-(17β-Hydroxy-17α-methyl-3-oxoandrost-4-en-7α-yl)heptyl]oxy]pentannitril
17β-Hydroxy-17α-methyl-7α-[9-[(4,4,5,5,5-pentafluorpentyl)sulfinyl]nonyl]androst-4-en-3-on
N-[9-(17β-Hydroxy-17α-methyl-3-oxoandrost-4-en-7α-yl)nonyl]methansulfonamid
7α-(9-Chlornonyl)-6β-hydroxy-17α-methyl-3-oxoandrost-4-en-17β-yl-acetat

10. Verwendung von Testosteronderivaten der allgemeinen Formel I zur Herstellung von Arzneimitteln, 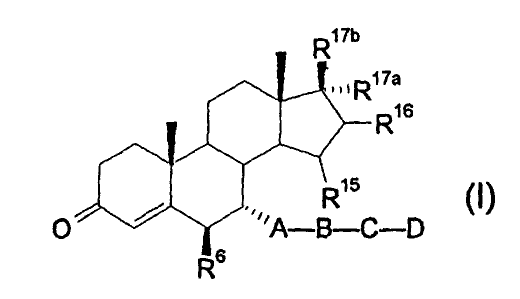 wobei in der allgemeinen Formel I
R⁶ ein Wasserstoffatom, eine Hydroxygruppe, eine C₁-C₁₀-Alkoxygruppe, eine C₁-C₁₀-Alkanoyloxygruppe oder ein Halogenatom darstellt,
R¹⁵ und R¹⁶ je ein Wasserstoffatom sind oder gemeinsam eine Bindung bilden,
R^{17a} eine C₁-C₄-Alkylgruppe, eine C₂-C₄-Alkinylgruppe, oder einen Rest der Formel CₙFₘHₒ darstellt, wobei n=1,2,3 oder 4, m>1 und m+o=2n+1 ist,
R^{17b} eine Hydroxygruppe, eine C₁-C₁₀-Alkoxygruppe oder eine C₁-C₁₀-Alkanoyloxygruppe ist,
A eine unverzweigte C₆-C₁₃-Alkylengruppe ist,
B ein Sauerstoffatom, eine Gruppierung -S(O)ₚ-, wobei p=0,1 oder 2 ist, eine Iminocarbonylgruppe -C(O)N(Y)-, eine Iminogruppe -N(Y)-, eine Carbonyliminogruppe N(Y)C(O)-, eine Sulfonyliminogruppe -N(Y)S(O)₂-, wobei Y ein Wasserstoffatom oder eine C₁-C₈-Alkylgruppe ist, eine Sulfonyloxygruppe -OS(O)₂-, eine Dimethylsilyloxygruppe -O-Si(CH₃)₂- oder eine Carbonylsulfanylgruppe -SC(O)- darstellt oder eine Bindung zwischen A und C darstellt oder zusammen mit C eine Bindung zwischen A und D bildet,
C eine Bindung zwischen B und D darstellt,oder zusammen mit B eine Bindung zwischen A und D bildet oder eine unverzweigte C₁-C₆-Alkylengruppe, eine Phenylengruppe, eine substituierte Phenylengruppe, eine Fünfring- oder Sechsring-Heteroarylengruppe, eine substituierte Fünfring- oder Sechsring-Heteroarylengruppe oder eine mit einem Phenylring kondensierte Fünfring- oder Sechsring-Heteroarylengruppe ist
und
D ein Wasserstoffatom, eine C₁-C₄-Alkylgruppe, eine Vinylgruppe, eine C₁-C₄-Alkoxygruppe, eine C₁-C₄-Alkoxycarbonylgruppe, eine Bis(C₁-C₄-alkoxycarbonyl)methylgruppe, eine Acetyl(C₁-C₄-Alkoxycarbonyl)methylgruppe, eine Cyangruppe, eine Carboxygruppe, eine Azidgruppe, eine Hydroxygruppe, ein Halogenatom, oder einen Rest der Formel CₙFₘHₒ darstellt, wobei n=1,2,3 oder 4, m>1 und m+o==2n+1 ist,
zur Langzeit-Antiandrogentherapie von Androgen-abhängigen Erkrankungen.

11. Verwendung gemäß Anspruch 10,
**dadurch gekennzeichnet, daß**
die Testosteronderivate zur Langzeittherapie des Prostatakarzinoms eingesetzt werden.

12. Verwendung gemäß Anspruch 10 oder 11,
**dadurch gekennzeichnet, daß**
die in den Ansprüchen 2 bis 9 näher beschriebenen Testosteronderivate eingesetzt werden.

13. Pharmazeutische Mittel enthaltend mindestens ein Testosteronderivat der allgemeinen Formel I gemäß der Ansprüche 1 bis 9 und in der Galenik übliche, physiologisch verträgliche Hilfs- und/oder Trägerstoffe.

## Claims

1. Testosterone derivatives of the general formula I 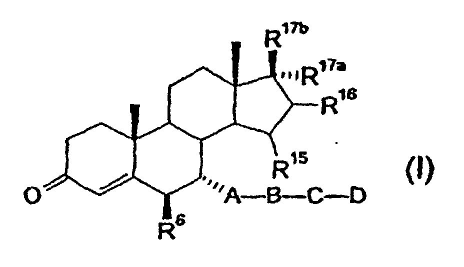 in which
R⁶ is a hydrogen atom, a hydroxyl group, a C₁-C₁₀-alkoxy group, a C₁-C₁₀-alkanoyloxy group or a halogen atom,
R¹⁵ and R¹⁶ are each a hydrogen atom or together form a bond,
R^{17a} is a C₁-C₄-alkyl group, a C₂-C₄-alkynyl group, or a radical of the formula CₙFₘHₒ where n=1, 2, 3 or 4, m>1 and m+o=2n+1,
R^{17b} is a hydroxyl group, a C₁-C₁₀-alkoxy group or a C₁-C₁₀-alkanoyloxy group,
A is an unbranched C₆-C₁₃-alkylene group,
B is an oxygen atom, an -S(O)ₚ- group, where p=0, 1 or 2, an iminocarbonyl group -C(O)N(Y)-, an imino group -N(Y)-, a carbonylimino group -N(Y)C(O)-, a sulphonylimino group -N(Y)S(O)₂-, where Y is a hydrogen atom or a C₁-C₈-alkyl group, a sulphonyloxy group -OS(O)₂-, a dimethylsilyloxy group -O-Si(CH₃)₂- or a carbonylsulphanyl group -SC(O)-, or is a bond between A and C, or forms together with C a bond between A and D,
C is a bond between B and D, or forms together with B a bond between A and D, or is an unbranched C₁-C₆-alkylene group, a phenylene group, a substituted phenylene group, a five-membered or six-membered heteroarylene group, a substituted five-membered or six-membered heteroarylene group, or a five-membered or six-membered heteroarylene group fused to a phenyl ring,
and
D is a hydrogen atom, a C₁-C₄-alkyl group, a vinyl group, a C₁-C₄-alkoxy group, a C₁-C₄-alkoxycarbonyl group, a bis(C₁-C₄-alkoxycarbonyl)methyl group, an acetyl(C₁-C₄-alkoxycarbonyl)methyl group, a cyano group, a carboxyl group, an azido group, a hydroxyl group, a halogen atom or a radical of the formula CₙFₘHₒ, where n=1, 2, 3 or 4, m>1 and m+o=2n+1.

2. Testosterone derivatives according to Claim 1, **characterized in that** R^{17a} is the methyl group, the ethyl group, the trifluoromethyl or pentafluoroethyl group.

3. Testosterone derivatives according to Claim 1 or 2, **characterized in that** R^{17b} is the hydroxyl group, a C₁-C₅-alkoxy group or a C₁-C₃-alkanoyloxy group.

4. Testosterone derivatives according to Claim 3, **characterized in that** R^{17b} is the hydroxyl, methoxy, ethoxy or acetyloxy group.

5. Testosterone derivatives according to any of Claims 1 to 4, **characterized in that** R⁶ is a hydrogen atom, the hydroxyl group or a halogen atom.

6. Testosterone derivatives according to any of Claims 1 to 5, **characterized in that** R¹⁵ and R¹⁶ are each a hydrogen atom.

7. Testosterone derivatives according to any of Claims 1 to 6, **characterized in that** the ABCD radical is 9-hydroxynonyl, 7-(acetylsulphanyl)-heptyl or 7-(4-cyanobutoxy)heptyl.

8. Testosterone derivatives according to any of Claims 1 to 6, **characterized in that** the five-membered or six-membered heteroaromatic systems in the radical C are pyrrole, thiophene, imidazole, thiazole, oxazole, triazole, thiadiazole, indole, benzoxazole, benzothiazole, pyridine or pyrimidine.

9. Testosterone derivatives according to any of Claims 1 to 8, **characterized in that** they are the following compounds:
7α-(9-chlorononyl)-17α-methyl-3-oxoandrost-4-en-17β-yl acetate
7α-(9-chlorononyl)-17β-hydroxy-17α-methylandrost-4-en-3-one
17β-hydroxy-7α-(9-iodononyl)-17α-methylandrost-4-en-3-one
17β-hydroxy-7α-(9-hydroxynonyl)-17α-methylandrost-4-en-3-one
7α-(10-chlorodecyl)-17β-hydroxy-17α-methylandrost-4-en-3-one
17β-hydroxy-7α-(11-hydroxyundecyl)-17α-methylandrost-4-en-3-one
7α-(11-bromoundecyl)-17β-hydroxy-17α-methylandrost-4-en-3-one
17β-hydroxy-17α-methyl-7α-[7-(phenylsulphanyl)heptyl]androst-4-en-3-one
17β-hydroxy-17α-methyl-7α-[9-[(4,4,5,5,5-pentafluoropentyl)sulphanyl]nonyl]androst-4-en-3-one
17β-hydroxy-17α-methyl-7α-[9-(phenylsulphanyl)nonyl]androst-4-en-3-one
7α-[9-[(5-chloropentyl)sulphanyl]nonyl]-17β-hydroxy-17α-methylandrost-4-en-3-one
17β-hydroxy-7α-[9-[(5-hydroxypentyl)sulphanyl]nonyl]-17α-methylandrost-4-en-3-one
7α-(9-azidononyl)-17β-hydroxy-17α-methylandrost-4-en-3-one
7α-[7-(acetylsulphanyl)heptyl]-17β-hydroxy-17α-methylandrost-4-en-3-one
17β-hydroxy-17α-methyl-7α-[7-[(4,4,5,5,5-pentafluoropentyl)sulphanyl]heptyl]androst-4-en-3-one
N-[7-(17β-hydroxy-17α-methyl-3-oxoandrost-4-en-7α-yl)heptyl]pentanamide
17β-hydroxy-17α-methyl-3-oxoandrost-4-en-7α-octanenitrile
5-[[7-(17β-hydroxy-17α-methyl-3-oxoandrost-4-en-7α-yl)heptyl]oxy]pentanenitrile
17β-hydroxy-17α-methyl-7α-[9-[(4,4,5,5,5-pentafluoropentyl)sulphinyl]nonyl]androst-4-en-3-one
N-[9-(17β-hydroxy-17α-methyl-3-oxoandrost-4-en-7α-yl)nonyl]methanesulphonamide
7α-(9-chlorononyl)-6β-hydroxy-17α-methyl-3-oxoandrost-4-en-17β-yl acetate.

10. Use of testosterone derivatives of the general formula I for producing medicaments 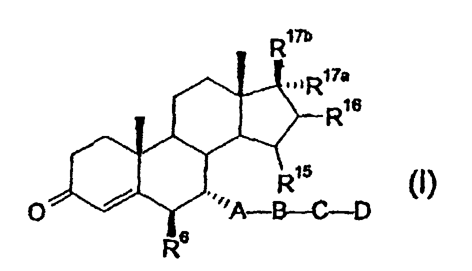 where in the general formula I
R⁶ is a hydrogen atom, a hydroxyl group, a C₁-C₁₀-alkoxy group, a C₁-C₁₀-alkanoyloxy group or a halogen atom,
R¹⁵ and R¹⁶ are each a hydrogen atom or together form a bond,
R^{17a} is a C₁-C₄-alkyl group, a C₂-C₄-alkynyl group, or a radical of the formula CₙFₘHₒ where n=1, 2, 3 or 4, m>1 and m+o=2n+1,
R^{17b} is a hydroxyl group, a C₁-C₁₀-alkoxy group or a C₁-C₁₀-alkanoyloxy group,
A is an unbranched C₆-C₁₃-alkylene group,
B is an oxygen atom, an -S(O)ₚ- group, where p=0, 1 or 2, an iminocarbonyl group -C(O)N(Y)-, an imino group -N(Y)-, a carbonylimino group -N(Y)C(O)-, a sulphonylimino group -N(Y)S(O)₂-, where Y is a hydrogen atom or a C₁-C₈-alkyl group, a sulphonyloxy group -OS(O)₂-, a dimethylsilyloxy group -O-Si(CH₃)₂- or a carbonylsulphanyl group -SC(O)-, or is a bond between A and C, or forms together with C a bond between A and D,
C is a bond between B and D, or forms together with B a bond between A and D, or is an unbranched C₁-C₆-alkylene group, a phenylene group, a substituted phenylene group, a five-membered or six-membered heteroarylene group, a substituted five-membered or six-membered heteroarylene group, or a five-membered or six-membered heteroarylene group fused to a phenyl ring,
and
D is a hydrogen atom, a C₁-C₄-alkyl group, a vinyl group, a C₁-C₄-alkoxy group, a C₁-C₄-alkoxycarbonyl group, a bis(C₁-C₄-alkoxycarbonyl)methyl group, an acetyl(C₁-C₄-alkoxycarbonyl)methyl group, a cyano group, a carboxyl group, an azido group, a hydroxyl group, a halogen atom or a radical of the formula CₙFₘHₒ/ where n=1, 2, 3 or 4, m>1 and m+o=2n+1,
for long-term antiandrogen therapy of androgen-dependent disorders.

11. Use according to Claim 10, **characterized in that** the testosterone derivatives are employed for the long-term therapy of prostate carcinoma.

12. Use according to Claim 10 or 11, **characterized in that** the testosterone derivatives defined in Claims 2 to 9 are employed.

13. Pharmaceutical compositions comprising at least one testosterone derivative of the general formula I according to Claims 1 to 9 and physiologically tolerated excipients and/or carriers customary in pharmaceutical technology.

## Revendications

1. Dérivés de testostérone de formule générale 1 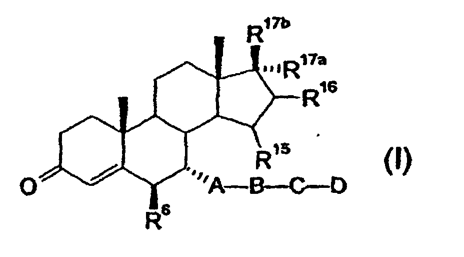 dans laquelle
R⁶ représente un atome d'hydrogène, un groupe hydroxy, un groupe alcoxy en C₁-C₁₀, un groupe alcanoyloxy en C₁-C₁₀ ou un atome d'halogène,
R¹⁵ et R¹⁶ représentent chacun un atome d'hydrogène ou forment conjointement une liaison,
R^{17a} représente un groupe alkyle en C₁-C₄, un groupe alcynyle en C₂-C₄ ou un radical de formule CₙFₘHₒ, dans laquelle n=1, 2, 3 ou 4, m et m+o=2n+1,
R^{17b} représente un groupe hydroxy, un groupe alcoxy en C₁-C₁₀ ou un groupe alcanoyloxy en C₁-C₁₀,
A représente un groupe alkylène en C₆-C₁₃ non ramifié,
B représente un atome d'oxygène, un groupement -S(O)ₚ-, dans lequel p=0, 1 ou 2, un groupe iminocarbonyle -C(O)N(Y)-, un groupe imino -N(Y)-, un groupe carbonylimino -N(Y)C(O)-, un groupe sulfonylimino -N(Y)S(O)₂-, où Y représente un atome d'hydrogène ou un groupe alkyle en C₁-C₈, un groupe sulfonyloxy -OS(O)₂-, un groupe diméthylsilyloxy -O-Si(CH₃)₂- ou un groupe carbonylsulfanyle -SC(O)- ou représente une liaison entre A et C, ou forme une liaison entre A et D conjointement avec C,
C représente une liaison entre B et D, ou forme une liaison entre A et D conjointement avec B, ou représente un groupe alkylène en C₁-C₆ non ramifié, un groupe phénylène, un groupe phénylène substitué, un groupe hétéroarylène à cinq chaînons cycliques ou six chaînons cycliques, un groupe hétéroarylène substitué à cinq chaînons cycliques ou six chaînons cycliques ou un groupe hétéroarylène à cinq chaînons cycliques ou six chaînons cycliques, condensé avec un noyau phénylique,
et
D représente un atome d'hydrogène, un groupe alkyle en C₁-C₄, un groupe vinyle, un groupe alcoxy en C₁-C₄, un groupe C₁-C₄-alcoxycarbonyle, un groupe bis(C₁-C₄-alcoxycarbonyl)méthyle, un groupe acétyl(C₁-C₄-alcoxycarbonyl)méthyle, un groupe cyano, un groupe carboxy, un groupe azide, un groupe hydroxy, un atome d'halogène, ou un radical de formule CₙFₘHₒ, dans laquelle n=1, 2, 3 ou 4, m>1 et m+o=2n+1.

2. Dérivés de testostérone selon la revendication 1, **caractérisés en ce que**
R^{17a} représente le groupe méthyle, le groupe éthyle, le groupe trifluorométhyle ou le groupe pentafluoroéthyle.

3. Dérivés de testostérone selon la revendication 1 ou 2, **caractérisés en ce que**
R^{17b} représente le groupe hydroxy, un groupe alcoxy en C₁-C₅ ou un groupe alcanoyloxy en C₁-C₃.

4. Dérivés de testostérone selon la revendication 3, **caractérisés en ce que**
R^{17b} représente le groupe hydroxy, méthoxy, éthoxy ou acétyloxy.

5. Dérivés de testostérone selon l'une quelconque des revendications 1 à 4, **caractérisés en ce que**
R⁶ représente un atome d'hydrogène, le groupe hydroxy ou un atome d'halogène,

6. Dérivés de testostérone selon l'une quelconque des revendications 1 à 5, **caractérisés en ce que**
R¹⁵ et R¹⁶ représentent chacun, un atome d'hydrogène.

7. Dérivés de testostérone selon l'une quelconque des revendications 1 à 6, **caractérisés en ce que**
le radical ABCD représente un 9-hydroxynonyle, un 7-(acétylsulfanyl)heptyle ou un 7-(4-cyanobutoxy)heptyle.

8. Dérivés de testostérone selon l'une quelconque des revendications 1 à 6, **caractérisés en ce que**
les composés hétéroaromatiques à cinq chaînons cycliques ou six chaînons cycliques du radical C sont le pyrrole, le thiophène, l'imidazole, le thiazole, l'oxazole, le triazole, le thiadiazole, l'indole, le benzoxazole, le benzothiazole, la pyridine ou la pyrimidine.

9. Dérivés de testostérone selon l'une quelconque des revendications 1 à 8, **caractérisés en ce qu'**ils représentent les composés suivants:
l'acétate de 7α-(9-chlorononyl)-17α-méthyl-3-oxoandrost-4-én-17β-yle
la 7α-(9-chlorononyl)-17β-hydroxy-17α-méthylandrost-4-én-3-one
la 17β-hydroxy-7α-(9-iodononyl)-17α-méthylandrost-4-én-3-one
la 17β-hydroxy-7α-(9-hydroxynonyl)-17α-méthylandrost-4-én-3-one
la 7α-(10-chlorodécyl)-17β-hydroxy-17α-méthylandrost-4-én-3-one
la 17β-hydroxy-7α-(11-hydroxyundécyl)-17α-méthylandrost-4-én-3-one
la 7α-(11-bromo-undécyl)-17β-hydroxy-17α-méthylandrost-4-én-3-one
la 17β-hydroxy-17α-méthyl-7α-[7-(phénylsulfanyl)heptyl]androst-4-én-3-one
la 17β-hydroxy-17α-méthyl-7α-[9-[(4,4,5,5,5-pentafluoropentyl)sulfanyl]nonyl]androst-4-én-3-one
la 17β-hydroxy-17α-méthyl-7α-[9-(phénylsulfanyl)nonyl]androst-4-én-3-one
la 7α-[9-[(5-chloropentyl)sulfanyl]nonyl]-17β-hydroxy-17α-méthylandrost-4-én-3-one
la 17β-hydroxy-7α-[9-[(5-hydroxypentyl)sulfanyl]nonyl]-17α-méthylandrost-4-én-3-one
la 7α-(9-azidononyl)-17β-hydroxy-17α-méthylandrost-4-én-3-one
la 7α-[7-(acétylsulfanyl)heptyl]-17β-hydroxy-17α-méthylandrost-4-én-3-one
la 17β-hydroxy-17α-méthyl-7α-[7-[(4,4,5,5,5-pentafluoropentyl)sulfanyl]heptyl]androst-4-én-3-one
le N-[7-(17β-hydroxy-17α-méthyl-3-oxoandrost-4-én-7α-yl)heptyl]pentanamide
le 17β-hydroxy-17α-méthyl-3-oxoandrost-4-ène-7α-octanenitrile
le 5-[[7-(17β-hydroxy-17α-méthyl-3-oxoandrost-4-én-7α-yl)heptyl]oxy]pentanenitrile
la 17β-hydroxy-17α-méthyl-7α-[9-[(4,4,5,5,5-pentafluoropentyl)sulfinyl]nonyl]androst-4-én-3-one
le N-[9-(17β-hydroxy-17α-méthyl-3-oxoandrost-4-én-7α-yl)nonyl]méthanesulfonamide
l'acétate de 7α-(9-chlorononyl)-6β-hydroxy-17α-méthyl-3-oxoandrost-4-én-17β-yle

10. Utilisation de dérivés de testostérone de formule générale I pour la préparation de médicaments, 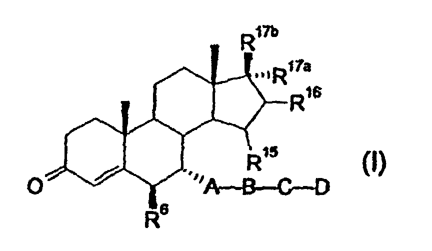 où dans la formule générale I
R⁶ représente un atome d'hydrogène, un groupe hydroxy, un groupe alcoxy en C₁-C₁₀, un groupe alcanoyloxy en C₁-C₁₀ ou un atome d'halogène,
R¹⁵ et R¹⁶ représentent chacun un atome d'hydrogène ou forment conjointement une liaison,
R^{17a} représente un groupe alkyle en C₁-C₄, un groupe alcynyle en C₂-C₄ ou un radical de formule CₙFₘHₒ, dans laquelle n=1, 2, 3 ou 4, m>1 et m+o=2n+1,
R^{17b} représente un groupe hydroxy, un groupe alcoxy en C₁-C₁₀ ou un groupe alcanoyloxy en C₁-C₁₀,
A représente un groupe alkylène en C₆-C₁₃ non ramifié,
B représente un atome d'oxygène, un groupement -S(O)ₚ-, dans lequel p=0,1 ou 2, un groupe iminocarbonyle -C(O)N(Y)-, un groupe imino -N(Y)-, un groupe carbonylimino -N(Y)C(O)-, un groupe sulfonylimino -N(Y)S(O)₂-, où Y représente un atome d'hydrogène ou un groupe alkyle en C₁-C₈, un groupe sulfonyloxy -OS(O)₂-, un groupe diméthylsilyloxy -O-Si(CH₃)₂- ou un groupe carbonylsulfanyle -SC(O)- ou représente une liaison entre A et C, ou forme une liaison entre A et D conjointement avec C,
C représente une liaison entre B et D, ou forme une liaison entre A et D conjointement avec B, ou représente un groupe alkylène en C₁-C₆ non ramifié, un groupe phénylène, un groupe phénylène substitué, un groupe hétéroarylène à cinq chaînons cycliques ou six chaînons cycliques, un groupe hétéroarylène substitué à cinq chaînons cycliques ou six chaînons cycliques ou un groupe hétéroarylène à cinq chaînons cycliques ou six chaînons cycliques, condensé avec un noyau phénylique,
et
D représente un atome d'hydrogène, un groupe alkyle en C₁-C₄, un groupe vinyle, un groupe alcoxy en C₁-C₄, un groupe C₁-C₄-alcoxycarbonyle, un groupe bis(C₁-C₄-alcoxycarbonyl)méthyle, un groupe acétyl(C₁-C₄-alcoxycarbonyl)méthyle, un groupe cyano, un groupe carboxy, un groupe azide, un groupe hydroxy, un atome d'halogène, ou un radical de formule CₙFₘHₒ, dans laquelle n=1, 2, 3 ou 4, m>1 et m+o=2n+1,
pour la thérapie anti-androgène à long terme de maladies androgène-dépendantes.

11. Utilisation selon la revendication 10, **caractérisée en ce que** les dérivés de testostérone sont utilisés pour la thérapie à long terme du carcinome de la prostate.

12. Utilisation selon la revendication 10 ou 11, **caractérisée en ce que** les dérivés de testostérone décrits plus en détail dans les revendications 2 à 4 sont utilisés.

13. Composition pharmaceutique comprenant au moins un dérivé de testostérone de formule générale I selon les revendications 1 à 9 et des auxiliaires et/ou supports physiologiquement acceptables, habituels en galénique.
